(19) Europäisches Patentamt European Patent Office Office européen des brevets

(11) **EP 4 663 111 A1**

(12) **EUROPEAN PATENT APPLICATION**

(43) Date of publication:
**17.12.2025 Bulletin 2025/51**

(21) Application number: **24181804.6**

(22) Date of filing: **12.06.2024**

(51) International Patent Classification (IPC):
**A61B 5/021** (2006.01)   **A61B 5/022** (2006.01)
**A61B 5/00** (2006.01)

(52) Cooperative Patent Classification (CPC):
**A61B 5/022; A61B 5/021; A61B 5/02141;**
**A61B 5/02225; A61B 5/6824; A61B 5/6843**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **FRANK, Katrin**
  **Eindhoven (NL)**
• **GERG, Maximilian Werner**
  **Eindhoven (NL)**
• **HOFFMANN, Benjamin**
  **5656AG Eindhoven (NL)**
• **PFEIFFER, Ulrich**
  **Eindhoven (NL)**
• **WIMMER, Julia**
  **Eindhoven (NL)**

(74) Representative: **Philips Intellectual Property &**
**Standards**
**High Tech Campus 52**
**5656 AG Eindhoven (NL)**

(54) **PRESSURE MEASURING DEVICE TO BE USED FOR DETERMINING A PHYSIOLOGICAL PARAMETER OF A SUBJECT**

(57)    The invention relates to a pressure measuring device 6 configured to surround a subject's body part 5, wherein the pressure measuring device comprises a) a pressure application element 88 configured to apply pressure to the body part, and b) a shell 4 arranged to be located between the pressure application element and the body part, when the pressure measuring device surrounds the body part 5. Furthermore, the pressure measuring device comprises c) a pressure sensing device comprising a fluid-filled pressure sensing pad 61 arranged in a pressure sensing area 72 extending, as measured in a proximal-distal direction 83, along a middle of an inner surface of the shell, wherein the pressure sensing device is configured to measure a pressure signal indicative of blood pulsations in the subject. The pressure measuring device allows for an accurate measurement of a physiological parameter like blood pressure, even if the subject is severely obese.

Fig. 7

**Description**

FIELD OF THE INVENTION

The invention relates to a pressure measuring device configured to surround a subject's body part, a pressure measuring method for measuring pressure of the subject by using the pressure measuring device, and an apparatus, method and computer program for determining a physiological parameter like the subject's blood pressure.

BACKGROUND OF THE INVENTION

In the article "Clinical Evaluation of a High-fidelity Upper Arm Cuff to Measure Arterial Blood Pressure during Noncardiac Surgery" by J. Briegel et al., Anesthesiology, volume 133, pages 997 to 1006 (2020), a blood pressure cuff is clinically evaluated that mainly differs from conventional oscillometric blood pressure cuffs in that it comprises a semi-rigid conical shell, a liquid-filled sensor pad and a pressure transducer connected to the sensor pad via a liquid-filled tube. By compressing a patient's upper arm by means of a pneumatic actuator encasing the shell, thus, a hydraulic coupling transferring tissue pressure pulses is established between the tissue and the pressure transducer via the sensor pad and the tube connecting the sensor pad with the pressure transducer. This hydraulic coupling has been found to allow for a recording of tissue pressure pulses with a high signal-to-noise ratio and hence for an accurate, "high-fidelity" blood pressure measurement based thereon.

SUMMARY OF THE INVENTION

It is an object of the present invention to provide a pressure measuring device that allows for an accurate measurement of a physiological parameter like blood pressure for obese subjects. It also is an object of the present invention to provide a corresponding pressure measuring method for measuring pressure of an obese subject by using the pressure measuring device, and an apparatus, method and computer program for determining a physiological parameter like the subject's blood pressure based on the measured pressure.

In a first aspect of the invention a pressure measuring device configured to surround a subject's body part (e.g., an upper arm, forearm, thigh, wrist or ankle of a human, or an animal's tail) is presented, wherein the pressure measuring device comprises a) a pressure application element configured to apply pressure to the body part, b) a shell arranged to be located between the pressure application element and the body part, to extend along the body part in a proximal-distal direction and to extend around the body part in a circumferential direction, when the pressure measuring device surrounds the body part, and c) a pressure sensing device configured to measure a pressure signal indicative of blood pulsations in the subject, the pressure sensing device comprising a fluid-filled pressure sensing pad arranged in a pressure sensing area extending along a middle of an inner surface of the shell, the middle being defined with respect to the proximal-distal direction.

By arranging a pressure sensing pad on an inner surface of a shell, wherein the shell is arranged to be located between the body part and the pressure application element, the sensed pressure signal can very accurately reflect the blood pulsations. In particular, a more accurate pressure signal can be acquired than with a conventional oscillometric blood pressure cuff, in which one and the same part is used both for applying pressure and for measuring pressure oscillations.

Obese subjects, and particularly severely obese subjects, have more volumetric body parts than leaner subjects. In particular, an amount of fat between the skin and inner vessels can be significantly higher for such subjects, which can lead to a decrease in signal quality of the measured pressure signal. Since more distal body parts generally tend to be leaner, one way to maintain a satisfactory measurement accuracy also for obese and severely obese patients is to use distal body parts for the measurement. It may be preferred, for instance, to use a forearm or a lower leg for the measurement. The pressure measuring device may thus preferentially be configured to surround a subject's forearm or lower leg, particularly a proximal part of a forearm or a distal part of a lower leg. It has been found that, when a distal body part is used for the measurement, a good measurement accuracy can particularly be achieved if the pressure sensing area extends along a middle of the inner surface of the shell, wherein the middle is defined with respect to the proximal-distal direction. At the same time, for measurements on proximal body parts such as an upper arm, still a sufficient measurement accuracy can be achieved when the pressure sensing area extends along the middle of the inner surface of the shell.

Pulsations of the brachial artery may be focused on in measurements on an upper arm. Instead, for measurements on a proximal part of a subject's forearm, tissue pressure pulses caused by pulsations of the radial, ulnar and interosseous arteries may be measured. For doing so, the pressure measuring device may be applied such that, during measurement, the pressure sensing area and hence the pressure sensing pad covers, in the circumferential direction, a position of the radial artery.

For measurements on a subject's distal lower leg, tissue pressure pulses caused by pulsations of the anterior and posterior tibial arteries may be measured. For doing so, the pressure measuring device may be applied such that, during measurement, the pressure sensing area and hence the pressure sensing pad covers, in the circumferential direction, a position of the posterior tibial artery.

The fluid in the pressure sensing pad is preferably gas, particularly air. Due to the higher compressibility of gases as compared to liquids, pressure waves generally propagate less well through gases than through liquids. Somewhat surprisingly, therefore, it has been found that also a gas-filled pressure sensing pad allows for a coupling to the interior of the subject's body part that is sufficiently strong to allow for a recording of tissue pressure pulses with good quality.

As compared to a liquid-filled pressure sensing pad, a gas-filled pressure sensing pad has the advantage that measurement errors due to shifts in hydrostatic pressures generated by movements of the subject can be avoided. If, for instance, the measurement is carried out with the measuring device surrounding a subject's upper arm, forearm or lower leg, and if it is necessary to reposition the subject, or at least the respective body part from a first position to a second position, wherein the orientation of the pressure sensing pad changes by the repositioning such that the extent of the pressure sensing pad, as viewed in the direction of the gravitational field, changes as well, then this will generally result in a changed pressure being measured. A correction of such changes in measured pressure is rather complicated.

The pressure application element is preferentially configured to apply pressure to the body part and the pressure sensing pad via the shell, when the shell is located between the pressure application element and the body part, wherein the pressure sensing device is configured to measure the pressure signal indicative of blood pulsations in the form of pressure pulses transmitted from the interior of the subject via the pressure sensing pad. The pressure measuring device preferentially comprises a pressure transducer, wherein the pressure pulses are transmitted via the pressure sensing pad and possibly further pressure guiding elements to the pressure transducer.

The pressure sensing device is preferentially configured to measure the pressure signal on the skin of the encased part of the subject, i.e., on the skin of the surrounded body part, while the pressure application element increases or decreases the applied pressure. Hence, the pressure measuring device can in principle be used in the same way as a blood pressure cuff of the known kind.

The pressure sensing pad being "fluid-filled" is to be understood such that the pressure sensing pad may be filled with a single fluid or a fluid mixture. The term "fluid" is therefore understood herein as including fluid mixtures. Moreover, fluids are understood herein as including liquids and gases.

Preferentially, the pressure sensing pad is air-filled. In that case, the production of the pressure measuring device does not require that the sensing pad is filled with any particular fluid, be it a liquid or some gas other than air. As a consequence, the costs for producing the pressure measurement device can be substantially reduced.

As described in more detail below, the pressure application element can comprise a fluid bag fillable with fluid for applying the pressure to the body part, wherein the fluid can be a gas or a liquid. With a fluid bag, pressure can be applied in a relatively uniform way to the body part. This allows for an accurate measurement of a physiological parameter like blood pressure.

The pressure measuring device is preferably configured to surround the distal body part with at least the shell extending around the respective body part in circumferential direction. Optionally, the pressure measuring device is configured to surround the body part also with the pressure application element.

As also described in more detail below, the shell preferentially is a kinking-proof shell, wherein the kinking-proof shell is located (or sandwiched) between the pressure application element, i.e., e.g., a fluid bag, and the body part, when the pressure measuring device surrounds the body part. The kinking-proof shell, which is relatively stiff, but flexible enough to adapt to various individual conical angles of respective body parts, avoids or at least significantly reduces the creation of wrinkles or kinks at the compression acting surface of the pressure application element, particularly of a fluid bag.

The shell can be regarded as allowing for a curled state and an uncurled state, wherein, in its curled state, it has a substantially cylindrical or slightly conical shape, and it can be brought from its curled state into its uncurled state by applying a mechanical force. The shell can insofar also be regarded as acting like a torsion spring. The pressure measuring device can be applied to surround the respective body part by uncurling the shell, to which the pressure application element may be attached, and then letting it curl around the body part. A completely uncurled state for shell may be hypothetical and only be considered for designing the shell. In reality, the shell may break if it were tried to uncurl it beyond some limit.

The cylindrical or slightly conical shape of the shell in its curled state may also be referred to as the shape of a cylinder that is tapered in proximal-distal direction. Preferably, when no external force acts upon the shell, the shell has the form of a cylinder that is tapered in proximal-distal direction at an angle between 4° and 5° relative to a non-tapered cylinder, particularly an angle of 4.5° relative to a non-tapered cylinder. The tapering is preferably a tapering from a proximal side to a distal side of the shell. The relatively steep "tapering angle" or "cone angle" of the shell has been found to be more suitable for obese patients, particularly for measurements on a forearm or lower leg. Specifically these body parts tend to be more conically shaped in obese patients. An accordingly steepened cone shape of the shell can lead to a more uniform pressure application to the body tissue and thus to a more accurate measurement. For non-obese patients, a tapering or cone angle of, for instance, only 3.2° may work very well already.

The dimensions of the shell may be chosen depending on a group of subjects and/or specific body parts for which the pressure measuring device is meant to be used, and hence may vary from shell to shell and from device to device. The dimensions of the shell may be defined with respect to a hypothetical completely uncurled shape, which may also be referred to as a flat condition. Moreover, the shape of the shell may be defined with respect to a circumferential direction and a proximal-distal direction, wherein the circumferential and the proximal-distal direction are perpendicular to each other. This nomenclature for the directions, particularly the proximal-distal direction, refers to a state where the pressure measuring device surrounds the respective body part, i.e., to its intended use. The proximal-distal direction may correspond to an axial direction of the shell in its curled state. The circumferential direction could also be viewed as a lateral direction, particularly in the uncurled state of the shell.

It is preferred that the shell has a dimension of 80 mm to 120 mm, preferably 100 mm, in the proximal-distal direction. Moreover, a dimension of the shell in the circumferential direction is preferentially at least 260 mm, more preferably at least 360 mm. These dimensions have particularly been found to be useful if applications are intended where the body part that the pressure measuring device is meant to surround during measurement is a patient's forearm or a distal part of a patient's leg, and if the patient has a forearm circumference of 260 mm to at least 360 mm. Generally, the ratio between the dimension of the shell in proximal-distal direction and the dimension of the shell in circumferential direction may be decreased as compared to a shell exclusively intended for lean patients and/or for measurements on an upper arm.

The pressure application element may have the same dimension as the shell in proximal-distal direction. In this way, the pressure application element, which may be a fluid bag surrounding the shell in circumferential direction, may apply a force and thus a pressure in radially inward direction that does not substantially vary in proximal-distal direction. At the same time, the dimension of the pressure measuring device in proximal-distal direction may not be larger than that of the shell.

That the pressure sensing area extends along a middle of the inner surface of the shell may broadly be understood such that the pressure sensing area covers the middle of the inner surface of shell in at least some region in circumferential direction. More particularly, it is preferred that the pressure sensing area is positioned substantially in the middle of the shell as viewed in proximal-distal direction. In other words, it is preferred that the region in which the pressure sensing area covers the middle of the inner surface of the shell has a substantial extent in circumferential direction. In particular, the pressure sensing area may cover the middle of the inner surface of the shell along a whole of a circumferential extent of the pressure sensing area. For instance, the pressure sensing area may be centered on the middle of the shell in proximal-distal direction, i.e., a midline of the pressure sensing area extending in circumferential direction may be aligned with the middle of the shell in proximal-distal direction. While, already in proximal-distal direction, the pressure sensing area may extend away from the middle of the shell, it may generally be elongated in circumferential direction, i.e., its circumferential extent may be larger, particularly substantially larger, than its proximal-distal extent.

An extent and a positioning of the pressure sensing area in the proximal-distal direction may be jointly defined relative to the shell. In such terms, it is preferred that a) a distance between a proximal edge of the pressure sensing area and a proximal edge of the shell and b) a distance between a distal edge of the pressure sensing area and a distal edge of the shell are both in a range between 20% and 40% of an entire dimension of the shell in the proximal-distal direction. The latter may also be referred to as a height of the shell. The proximal edge of the shell and the distal edge of the shell may particularly refer to an outer proximal edge and an outer distal edge of the shell, respectively. It is particularly preferred that a) the distance between the proximal edge of the pressure sensing area and the proximal edge of the shell and b) the distance between the distal edge of the pressure sensing area and the distal edge of the shell are the same, specifically 35% of the entire dimension of the shell in the proximal-distal direction, i.e., the entire height of the shell. This may hold along a whole circumferential extent of the pressure sensing area. More generally, a) the distance between the proximal edge of the pressure sensing area and the proximal edge of the shell and/or b) the distance between the distal edge of the pressure sensing area and the distal edge of the shell may vary in circumferential direction.

Generally independently from the positioning of the pressure sensing area, but particularly for the preferred positioning indicated above, it is preferred that the pressure sensing area has the following dimensions.

In relative terms, the pressure sensing area preferentially has a dimension between 45% and 95%, more preferably between 59% and 83%, of an inner circumference of the shell in the circumferential direction and a dimension between 15% and 40%, preferentially a dimension between 30% and 40%, of the entire shell height in the proximal-distal direction. The dimension may be measured with respect to a flat condition of the shell. In the flat condition, the pressure sensing area may be shaped generally rectangular, possibly slightly curving in lateral direction to follow a curvature that also the shell may have in its flat condition to allow for a conical shape when curled-up. The inner circumference of the shell relative to which the dimension of the pressure sensing area may be given may correspond to a maximum circumference of a respective body part for which the pressure measuring device is designed (maximum of working range).

In absolute terms, the pressure sensing area may preferentially have, as may be measured with respect to a flat condition of the shell, a dimension between 70 mm and 215 mm in the lateral or circumferential direction and a dimension between 25 mm and 45 mm, ideally between 30 and 40 mm, in the proximal-distal direction. The above-indicated relative dimension of the pressure sensing area of 59% to 83% of an inner circumference of the shell in the circumferential direction may particularly hold if its absolute dimension is 215 mm in the lateral, i.e., circumferential direction.

It should be emphasized that the above indicated dimensions of the pressure sensing area are not the only possible ones. For instance, a sufficiently accurate pressure measurement may also be possible with a pressure sensing area having a dimension of below 70 mm in lateral direction and 40 mm in proximal-distal direction. Hence, in particular, the dimension of the pressure sensing area in the lateral dimension may also be substantially smaller than indicated above. With such smaller sensing areas, however, a noticeable dependence of the pressure measurement on an orientation with which the shell is applied to the respective body part has been observed. In order to minimize this dependence and thereby increase the measurement accuracy, it has been found advantageous to position the pressure sensing area in the shell such that it is displaced, in lateral direction, from an arterial shell positioning line of the shell, the arterial shell positioning line being indicated by one or more visible marks that are provided on the pressure measuring device to indicate how the shell should be applied to the respective body part. When applied optimally, the arterial shell positioning line is aligned with a respective artery of the body part. For instance, in a pressure measuring device to be used on a subject's forearm, the arterial shell positioning line may preferably be aligned with the radial artery, and in a pressure measuring device to be used on a subject's lower leg, the arterial shell positioning line may preferably be aligned with the posterior tibial artery. With a sufficient lateral displacement of the center of the pressure sensing area relative to the arterial shell positioning line, a tolerance against relatively large misplacements of the pressure measuring device and hence between the arterial shell positioning line and a respective artery can be achieved. For instance, with a lateral displacement of the center of the pressure sensing area from the arterial shell positioning line by -60° (measured as an angular offset with respect to an imaginary rotational axis coinciding with the axis of a cylindrical or conical shape of a shell when in a curled-up state), misplacements of the pressure measuring device by up to +60° or more can become tolerable, and vice versa.

In principle, the above described dependence of the pressure measurement on the positioning of the shell, and particularly the pressure sensing area, relative to the respective body part also persists for pressure sensing areas with a larger dimension in the lateral direction, although the dimensions and the positioning described above have been found to largely mitigate any measurement inaccuracies arising therefrom. Nevertheless, to further avoid such consequences, it may generally also be preferred to dimension the pressure sensing area in the lateral direction yet larger. In principle, the dimension of the pressure sensing area in lateral direction may be chosen arbitrarily, in particular up to 100 % of a circumference of the respective body part, particularly a maximum circumference for which the pressure measuring device is designed (maximum of working range).

The pressure sensing area preferentially extends into an end portion of the shell, i.e., an end portion as viewed in circumferential direction and thus a circumferential end portion. The end portion is preferentially tapered. That is to say, if viewing the shell in its hypothetical completely uncurled state, the proximal and distal edges of the shell are tapered towards a lateral edge, wherein this lateral edge is the lateral edge of the end portion. The height of the shell in proximal-distal direction hence becomes smaller towards the lateral edge, particularly in the end portion. The tapered end portion may be defined as beginning, as viewed from the center to the lateral edge, at the lateral position where the height of the shell begins to decrease.

A distance between the pressure sensing area and an edge of the end portion of the shell is preferentially chosen to ensure that the end portion of the shell does not touch and thereby disturb the pressure sensing area during measurement under any circumstances, and may for this purpose particularly be at least 5 mm. By arranging the pressure sensing area and hence the pressure sensing pad in this way, the pressure measuring device can be applied to the respective body part in a smooth sliding manner, while still allowing for an accurate pressure measurement because mechanical contacts/disturbances of the pressure sensing pad are avoided. The edge of the end portion may be referred to as a lateral edge, and may extend in a proximal-distal direction.

As already indicated above, the distance between the pressure sensing area and the distal edge of the shell may be, for instance, within a range from 20% to 40% of the entire height of the shell from its outer distal edge to its outer proximal edge. In a preferred embodiment, as also indicated above, the distance between the outer distal edge of the pressure sensing area and the outer distal edge of the shell is 35%.

The pressure application element and the shell are preferentially configured such that, when both are applied to surround a subject's body part, the pressure application element is configured to apply pressure to the body part via the shell. Upon increasing application of pressure, a diameter of the shell may decrease by virtue of its circumferential end portions successively sliding further over each other such that their respective edges have an increasing distance to each other in circumferential direction. Without the body part being surrounded, a cross-section through the shell in a plane perpendicular to the proximal-distal, or axial, direction may remain generally circular even if pressure is applied to the shell from the outside. That is to say, in the cross-section, the pressure application may result in the shell forming circles with successively decreasing diameter. This may still be the case in the presence of a body part being surrounded, if the body part has a similar shape as the shell or at least is sufficiently deformable to assume such a shape, as may be the case for an upper arm, for instance, where the bones are normally surrounded by a sufficient amount of deformable muscle and/or fat tissue. However, in typically leaner body parts, and particularly the forearm or lower leg, the bones are more prominent even in obese patients. This can hinder a circular shrinkage of the shell, and thus can detrimentally affect the uniform pressure application desired for accurate measurements. In particular, the ulnar bone in the forearm as well as the tibia in the lower leg can inhibit an ideal adaption of the shell to the respective body part.

In view of the above considerations, it has been realized that it can be preferred that the shell is segmented in circumferential direction, wherein at least one segment of the shell is thinner and/or more flexible than at least one other segment or a remaining portion of the shell. The at least one thinner and/or more flexible segment is preferably positioned such that it can be placed on a respective prominent bone of the body part, such as the ulnar bone or tibia, if the pressure sensing pad is placed at, i.e., at least covers, a circumferential position of a respective artery. A solution particularly suitable for patients with a strong pronounced ulnar bone could be a shell comprising multiple segments. The segments may differ in their thickness and/or flexibility. The one or more thinner and/or more flexible shell segments allow to surround a respective bone for better fixation and adaptation of the shell to the curvature of the respective body part.

The pressure sensing device can comprise a tube for guiding pressure from the pressure sensing pad to a pressure transducer. In particular, the pressure sensing device may comprise a pressure transducer housing in which the pressure transducer is located. The tube may thus be adapted for guiding pressure from the pressure sensing pad to the pressure transducer housing and thereby to the pressure transducer. The tube may also be referred to as a pressure guiding tube. The pressure transducer, particularly the pressure transducer housing with the pressure transducer, is preferably attachable to an outer surface of the pressure measuring device. Additionally or alternatively, they may be mountable to an external mount that is separate from the pressure measuring device.

It is preferred that the tube is gas-filled. Hence, it is preferred that not only the pressure sensing pad is filled with gas as indicated above, but also the tube. This has the considerable advantage that the relative height between the point where the tube enters the pressure sensing pad and the point where the pressure transducer is located, particularly where the tube enters the pressure transducer housing, does not need to be taken into account in the pressure measurement. In particular, a zeroing of the pressure transducer at the height of the sensor pad, as is conventionally done when using a liquid-filled pressure sensing pad connected to the pressure transducer via a liquid-filled tube, is no longer necessary. It will be understood that the weight of the gas in the tube and hence any pressure offset resulting from a difference in height between the point where the tube enters the pressure sensing pad and the point where the pressure transducer is located, particularly where the tube enters the pressure transducer housing, will be negligible for all practical purposes.

The gas in the tube may be the same as the gas in the pressure sensing pad. The tube may then be connected to the pressure sensing pad such that the gas freely communicates therebetween. For instance, the tube may be stuck, glued, welded or solvent-bonded into a hole of the pressure sensing pad, or into one side of a connector whose other side is open towards the interior of the pressure sensing pad, wherein the connector may be fixed in the respective wall of the pressure sensing pad.

Preferably, there is no dedicated connector connecting the tube with the pressure sensing pad. Instead, it may be preferred that the tube and the pressure sensing pad are formed as a single, continuous piece of plastic.

Also the pressure transducer housing is preferentially filled with gas and may, in particular, be filled with the same gas as the pressure sensing pad. Hence, in particular, all of the pressure sensing pad, the pressure guiding tube and the pressure transducer housing may be air-filled.

Like the connection between the tube and the pressure sensing pad, also the connection between the tube and the pressure transducer housing may be realized using a dedicated connector or not. While as dedicated connector, for instance, a Luer connector may be used, it may be preferred that also the tube and the pressure transducer housing are connected without a dedicated connector, such as by sticking, gluing, welding or solvent-bonding the tube into a hole in the pressure transducer housing. In fact, the whole pressure sensing device may be jointly manufactured by machine-driven plastics materials assembly.

Dedicated connectors connecting the tube with the pressure sensing pad and/or the pressure transducer housing can be a simple, practical solution, but bear the risk that users inadvertently open the respective connection and in this way damage the pressure sensing device, which otherwise may be a closed system. Therefore, it is preferred to establish a smooth inner fluid path throughout the whole pressure sensing device, i.e., to avoid any stenosis-like reductions in inner diameter, by machine-driven plastics materials assembly. Indeed, it has been found that such a smooth fluid path can also be advantageous regarding measurement accuracy if the fluid is a gas such as air.

Preferentially, the pressure sensing pad has a relatively high flexibility, but nevertheless is physically robust. In particular, its walls may be formed from a foil that is as thin as possible as long as it is at the same time robust enough to potential physical damage. The pressure guiding tube is preferentially fairly thick (e.g., with an outer diameter of 2.0 mm and an inner diameter of 1.2 mm), i.e., may be a usual invasive blood pressure extension tubing that is as short as possible and as long as required. A fluid volume, particularly air volume, in the pressure sensing pad, the tube and/or the pressure transducer housing is preferably kept as low as possible in order to avoid significant damping by the fluid itself, but as high as required to avoid any pressure pulse degradation on the way from the pressure sensing pad to the pressure transducer.

When all of the pressure sensing pad, the connecting pressure tube and the pressure transducer housing are filled with air, the coupling between the interior of the body part and the pressure transducer may be viewed as having a substantial pneumatic component. It has been found that even in this case still an accurate measurement of a physiological parameter like blood pressure in the very low range, e.g., a mean arterial blood pressure (MAP) of 40 mmHg in extremely obese patients, can be carried out, particularly at a maximum of the measured tissue pressure pulses (TPPmx, as further explained below) of about very low to 1 mmHg with visible tissue pressure pulse waveforms exhibiting a dicrotic notch. Moreover, in this case no parts of the pressure measuring device need to be filled with liquid during production, which substantially reduces production costs.

Generally, having elements of the pressure sensing device filled with gas instead of liquid will lead to lower maxima in measured tissue pressure pulses (TPPmx) and quantities derived therefrom (e.g., TFmx, explained further below). However, any liquid-filled system is also more sensitive to capturing noise than a corresponding gas-filled system (e.g., vibrations, movement artifacts). Such noise could especially be transmitted through the tube connecting the pressure sensing pad with the pressure transducer, since this is preferably not fixed, in order to adjust to a large range of sizes of body parts to be used for the measurement. The signal-to-noise ratio of a pressure sensing device with gas-filled elements can therefore be better than the one of a liquid-filed pressure sensing device.

Despite the benefits of gas-filled and particularly air-filled components of the pressure sensing device, it remains an option that some or all of the components are liquid-filled. In particular, the pressure sensing pad and the pressure guiding tube may be air-filled while the pressure transducer housing is filled with liquid, without risking a substantial decrease in measurement accuracy. Also, a sufficient measurement accuracy may still be achieved if the pressure sensing pad, the pressure guiding tube and the pressure transducer housing are filled with liquid. Where a liquid is used to fill one or more components of the pressure measuring device, this liquid may preferentially be silicone oil or glycerol.

Structural features of the pressure sensing device, particularly a material and dimensions of the pressure sensing pad, the pressure guiding tube and the pressure transducer housing as well as a connection between any two of the three, may be chosen irrespectively of the fluid chosen for the filling, i.e., as pressure transmitting medium. Where a risk of liquid spilling from a liquid-filled component into a gas-filled component of the pressure sensing device could occur, a pressure transmitting membrane may be provided between the two components. For instance, liquid spilling from a liquid-filled pressure transducer housing into an air-filled pressure guiding tube may be avoided by connecting the tube to the pressure transducer housing using a connector comprising a pressure-transmitting membrane forming an interface between the air in the tube and the liquid in the pressure transducer housing.

As long as one or more of the pressure sensing pad, the pressure guiding tube and the pressure transducer housing are filled with air, the pressure sensing device preferentially further comprises a valve for venting the respective one or more elements. By equalizing the pressure in the one or more elements to atmospheric pressure via the valve before a pressure measurement is initiated, the pressure measuring device can be used in regions with differing atmospheric pressures, i.e., different heights above sea level can be zeroed appropriately. For instance, if, after having carried out a blood pressure measurement at an altitude of 2000 m above sea level with the device, the device is to be used for a blood pressure measurement at sea level, the pressure difference between the air-filled elements of the device and the environment will have increased, leading to a decreased air volume in the respective one or more elements. This can be adjusted by using the valve for venting the one or more elements.

Preferentially, the tube and the pressure sensing pad are filled with air, and the valve is arranged on the tube, i.e., may be part of the pressure guiding tube connecting the pressure sensing pad with the pressure transducer housing. Optionally, the tube extends through the pressure transducer housing, wherein the valve is arranged on the part of the tube extending beyond the pressure transducer housing. Instead of the tube guiding the pressure from the pressure sensing pad to the pressure transducer housing extending through the pressure transducer housing, it is also possible that a further tube extends from the pressure transducer housing, wherein then the valve may be arranged on, or be part of, this further tube. This may particularly be preferred in case all of the pressure sensing pad, the tube and the pressure transducer housing are filled with air.

With the valve being arranged on a tube, or tube part, extending from the pressure transducer housing, i.e., extending further away from the pressure sensing pad, then venting of the pressure sensing device may be carried out from a distance. For instance, an end of the tube or tube part that is opposite to its end facilitating its connection to the pressure transducer housing may be open to the atmosphere, wherein the valve may be operable to trap an air volume inside the tube, or tube part, and/or open it to the atmosphere. If all of the pressure sensing pad, the pressure transducer housing and the one or more tubes are filled with air, the air volume may be common to all of them, i.e., all of them may be selectively vented and/or closed-off collectively.

The valve may be a stopcock, a snap-in valve, or a touch valve, for instance. Moreover, the valve may be operable manually or automatically. In particular, the valve may per default be open, wherein it may be closed manually or automatically after zeroing of the pressure sensing system.

In fact, a tube for guiding the pressure from the pressure sensing pad to the pressure transducer is not needed in all embodiments. In particular, the pressure sensing device may comprise a wired or wireless pressure transducer located within the pressure sensing pad or in between the pressure sensing pad and the shell, particularly between the pressure sensing pad and the inner surface of the shell. The wired or wireless pressure transducer may be configured to transmit sensed pressure signals wired or wirelessly to an external processor. Also a separate housing for the pressure transducer would in this case not be needed. In order to allow for a redundant pressure measurement and thereby potentially avoid measurement errors, both an internal and an external pressure transducer may be used, wherein the internal pressure transducer may be a wired or wireless pressure transducer located within the pressure sensing pad or in between the pressure sensing pad and the shell, and the external pressure transducer may be connected to the pressure sensing pad via a pressure guiding tube as indicated further above, for guiding a pressure signal to an exterior of the shell.

In an example, a compressible mesh is arranged within the pressure sensing pad. This allows to prevent a closure of the pressure guiding tube at a connection to the pressure sensing pad. Moreover, the mesh, which may also be regarded as a spacer fabric, may be integrated in the pressure sensing pad such that it creates a predefined height and volume of the pressure sensing pad, which may, like the valve indicated above, be advantageous to use the pressure measuring device in regions with different atmospheric pressure.

The mesh is preferably strong and/or thick enough to reproducibly establish a complete air-filling of the pressure sensing pad with the pressure sensing device opened to the atmosphere in a non-attached state of the pressure measuring device, particularly the shell. On the other hand, the mesh is preferably weak enough to not compromise pressure sensing by creating an internal counter-pressure in the pressure sensing pad, i.e., the mesh is preferentially compressible at relatively low attachment pressures already, e.g., 5 mmHg. It may be glued to an interior surface of walls of the pressure sensing pad, preferably with a flexible glue and to an interior surface of both an inner and an outer wall of the pressure sensing pad, wherein "inner" and "outer" may be understood as referring to a radial direction of the shell in its curled-up state, i.e., a direction perpendicular to the lateral and the proximal-distal direction. In use, the inner and the outer wall of the pressure sensing pad would therefore be parallel to an inner surface of the shell.

The mesh also allows to prevent the formation of non-gas-filled compartments within the pressure sensing pad. This allows for an improved measurement of the pressure signal, which finally can lead to a further improved determination of the physiological parameter based on the improved pressure signal. The mesh could equivalently be referred to as a woven structure. The mesh is preferentially a three-dimensional mesh. For instance, it can comprise two or more layers separated from each other in a direction perpendicular to the layers.

The pressure sensing pad may comprise walls made of a weldable foil. The material of the foil may be chosen depending on the fluid with which the pressure sensing pad is filled. Hence, in particular, an air-tight foil may be chosen. For instance, an airtight weldable foil, preferably with a thickness of 50 $\mu$m to 200 $\mu$m, such as a thermoplastic polyurethane (TPU) film, an ethylene-vinyl acetate (EVA) foil or the like may be chosen to form the walls of the pressure sensing pad.

In a further aspect of the present invention, an apparatus for determining a physiological parameter of a subject is presented, wherein the apparatus comprises a) the pressure measuring device for measuring the pressure signal of the subject over a time, wherein the measured pressure signal is indicative of blood pulsations, and b) a processor configured to determine the physiological parameter based on the measured pressure signal. Preferentially, the measured pressure signal comprises a plurality of pressure pulses. Moreover, the physiological parameter can be determined continuously, i.e. several subsequent physiological parameter measurements can be carried out, or it can be determined non-continuously, i.e. for instance one time.

The processor can be configured to determine, for a respective pressure pulse of the plurality of pressure pulses, at least one feature that characterizes the respective pressure pulse, to determine, for the respective pressure pulse, a physiological parameter determination value based on the determined at least one feature such that for several pressure pulses, which are present at different times, several physiological parameter determination values are determined, wherein the several physiological parameter determination values determined for the several pressure pulses and hence for several times form a physiological parameter determination curve, and to determine the physiological parameter based on the physiological parameter determination curve. For instance, the physiological parameter can be the blood pressure, the respiration rate or another physiological parameter.

The at least one feature can directly characterize the respective pressure pulse or indirectly characterize the respective pressure pulse. In the latter case the respective pressure pulse is processed for determining a feature determination pulse and the at least one feature is determined based on the determined feature determination pulse. This will be explained in more detail further below. The processor can be configured to determine, for each pressure pulse of all or of only some, for instance, at least 5 or at least 10, of the plurality of pressure pulses, the at least one feature that characterizes the respective pressure pulse.

The processor can be adapted to process the several physiological parameter determination values, which are obtained for the several pressure pulses, for obtaining a continuous physiological parameter determination curve. For instance, an interpolation can be applied and optionally also a smoothing.

In an embodiment, the processor is configured to, in order to determine for a respective pressure pulse the at least one feature, provide a feature determination pulse based on the respective pressure pulse, and determine at least one of the following features i) the difference (TPP) between the feature determination pulse's maximum systolic pressure (TPsys) and the feature determination pulse's pressure at the end-diastolic point (TPdia), ii) the area (TPA+.top50) enclosed by an upper part of the feature determination pulse, wherein a) the upper end of the upper part is at the feature determination pulse's maximum systolic pressure (TPsys) and b) the lower end of the upper part is between the feature determination pulse's maximum systolic pressure (TPsys) and a pressure value which corresponds to a mean (TPcl) of the measured pressure (TP), iii) the duration (t(pulse)) of the respective feature determination pulse, iv) the area (TPA/TPA.norm) of the respective feature determination pulse, and v) the width at half maximum (W50) of the respective feature determination pulse. It has been found that by using at least one of these features the determination of the physiological parameter can be further improved.

The feature determination pulse for a respective pressure pulse can be obtained, for instance, by subtracting a mean measured pressure (TPcl) from the respective pressure pulse. However, the feature determination pulse for a respective pressure pulse can also be determined in another way. It can also directly be the respective pressure pulse, i.e. the respective measured pressure pulse. If the feature determination pulse for a respective pressure pulse has been obtained by subtracting a mean measured pressure (TPcl) from the respective pressure pulse, for determining the area TPA+.top50 the pressure value, which corresponds to the mean (TPcl) of the measured pressure (TP), would be zero.

The difference (TPP) between the feature determination pulse's maximum systolic pressure and the feature determination pulse's pressure at the end-diastolic point preferentially is the difference between the maximum measured pressure and the minimum measured pressure of the respective pressure pulse, i.e. the difference between the tissue pressure (TP), i.e. the measured pressure on the skin, of the respective pressure pulse at a maximum systolic point and the tissue pressure (TP) at an end diastolic point of the respective pressure pulse. The duration t(pulse) of the respective feature determination pulse preferentially corresponds to the time difference between an end-diastolic point and the following end-diastolic point for the respective pressure pulse. Moreover, the area TPA is preferentially the area under the curve of the respective feature determination pulse from an end diastolic point to the following end diastolic point. The area can be a normalized area and is then named "TPA.norm". For instance, the area TPA can be normalized by scaling it to the difference between the maximum pressure and the minimum pressure of the respective feature determination pulse, in order to determine TPA.norm. The width W50 at half maximum corresponds to the width at 50 percent of the difference between the maximum pressure and the minimum pressure of the respective feature determination pulse. Thus, it is the width at 50 percent of the difference between the pressure of the feature determination pulse at the maximum systolic point and the pressure of the feature determination pulse at an end diastolic point.

In a preferred embodiment the processor is configured to determine the area TPA+.top50 enclosed by the upper part of the feature determination pulse such that the lower end of the upper part is at a pressure value which corresponds to half of the pressure distance (TPP+) between the feature determination pulse's maximum systolic pressure (TPsys) and the pressure value which corresponds to the mean (TPcl) of the measured pressure (TP).

Moreover, in a preferred embodiment the processor is configured to determine the physiological parameter determination values (TPWP_M) and hence form the physiological parameter determination curve (TPW_M-curve) only based on the feature being the difference (TPP) between the feature determination pulse's maximum systolic pressure (TPsys) and the feature determination pulse's pressure at the end-diastolic point (TPdia). Thus, in this embodiment, besides TPP, no other of the above mentioned features is used for determining the physiological parameter determination values. It has been found that this allows for a further increased accuracy of determining the physiological parameter with relatively low computational efforts.

In a further improved embodiment the processor is configured to determine the physiological parameter determination values (TPWP_M) and hence form the physiological parameter determination curve (TPW_M-curve) only based on a) the feature being the difference TPP between the feature determination pulse's maximum systolic pressure (TPsys) and the feature determination pulse's pressure at the end-diastolic point (TPdia) and b) the feature being the area TPA+.top50 enclosed by the upper part of the feature determination pulse. Thus, in this embodiment, besides TPP and TPA+.top50, no other of the above mentioned features is used for determining the physiological parameter determination values. It has been found that this allow for an even further increased accuracy of determining the physiological parameter, wherein still the computational efforts can be relatively low.

Preferentially, the processor is configured to determine a position of a maximum of the physiological parameter determination curve (TPW_M-curve) and to determine the physiological parameter, particularly the blood pressure, based on the determined position and the measured pressure (TP). In particular, the processor can be configured to determine the blood pressure based on the mean (TPcl) of the measured pressure (TP) at the determined position of the maximum. Determining the physiological parameter based on the determined maximum of the physiological parameter determination curve and depending on the measured pressure on the skin, i.e. depending on the tissue pressure, allows to further increase the accuracy of determining the physiological parameter, particularly the blood pressure.

Preferentially the processor is configured to provide a function which receives as input the at least one feature of a respective pressure pulse and which outputs a respective physiological parameter determination value which forms, together with the physiological parameter determination values determined for the other pressure pulses, the physiological parameter determination curve. The function has at least one parameter which can be determined by calibration, wherein by another measurement gold standard physiological parameters are determined very accurately and the at least one parameter is determined such that the apparatus yields the very accurately measured gold standard physiological parameters with high statistical precision and accuracy. For instance, by invasive means reference blood pressure values are determined very accurately and at least one parameter is determined such that the apparatus yields the very accurately invasively measured blood pressure values with high statistical precision and accuracy. In particular, for the calibration measurement pairs of simultaneously recorded invasive and noninvasive blood pressure values from an adequate number of individuals in different hemodynamic conditions are used. It should be noted that the calibration is preferentially only carried out in a development phase, i.e. not during an actual blood measurement procedure. The calibration can be carried out separately for different shell sizes or for different groups of shell sizes. For instance, for different shell sizes or groups of shell sizes, different parameters of the function can be determined by calibration, i.e. for each shell size or for each group of shell sizes a respective at least one parameter can be determined.

In an embodiment the processor is configured to control the pressure applicator such that it increases the applied pressure with a first rate in a pre-measurement time period which is followed by the measurement time period in which the applied pressure is increased with a second rate, wherein the first rate is larger than the second rate. Thus, an applied pressure, at which the measurement time period should start, can be reached relatively fast, thereby allowing for a further reduction of the overall time needed for measuring the blood pressure.

In an embodiment the processor is configured to control the pressure actuator such that at the end of the pre-measurement time period the measured pressure is within a range of 15 to 30 mmHg. This ensures that during the measurement time period the pressure on the skin, i.e. the tissue pressure, is measured over a sufficiently large pressure range, which allows for an accurate and precise measurement of the physiological parameter.

It should be noted that the pressure at the end of the pre-measurement time is not the pressure which is applied in between two subsequent blood pressure measurements, particularly before the pre-measurement time period starts. This pressure could be named "attachment pressure" and preferentially does not exceed 15 mmHg.

In another aspect of the present invention a pressure measuring method for measuring pressure of a subject is presented, wherein the pressure measuring method comprises a) arranging a pressure measuring device as defined above around a body part of the subject, b) applying pressure to the body part by a pressure application element of the pressure measuring device, and c) measuring a pressure signal of the subject, wherein the measured pressure signal is indicative of blood pulsations, by the pressure sensing device of the pressure measuring device.

In a further aspect of the present invention a method for determining a physiological parameter of a subject is presented, wherein the method comprises a) measuring a pressure signal of the subject over a time, wherein the measured pressure signal is indicative of blood pulsations, as defined above, and b) determining the physiological parameter based on the measured pressure signal by a processor.

In another aspect of the present invention a computer program for determining a physiological parameter of a subject is presented, wherein the computer program comprises program code means for causing the apparatus for determining a physiological parameter as defined above to carry out the steps of the method for determining a physiological parameter of a subject as defined above.

It shall be understood that the pressure measuring device of claim 1, the apparatus for determining a physiological parameter of claim 12, the pressure measuring method of claim 13, the method for determining a physiological parameter of claim 14 and the computer program for determining a physiological parameter of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplary an embodiment of an apparatus for determining a physiological parameter of a subject, which comprises a pressure measuring device, in a situation in which a pressure cuff of the pressure measuring device is inflated,

Fig. 2 shows schematically and exemplary a shell of the pressure measuring device encasing a body part like an upper arm of the subject,

Fig. 3A shows schematically and exemplarily the pressure measuring device encasing a proximal part of a forearm of the subject,

Fig. 3B shows schematically and exemplarily a cross-sectional view of the shell of the pressure measuring device encasing the proximal part of the forearm of the subject,

Fig. 4A shows schematically and exemplarily the pressure measuring device encasing a distal part of a lower leg of the subject,

Fig. 4B shows schematically and exemplarily a cross-sectional view of the shell of the pressure measuring device encasing the distal part of the lower leg of the subject,

Fig. 5 shows schematically and exemplary the apparatus in a situation in which the pressure cuff is deflated,

Fig. 6 shows schematically and exemplarily the pressure measuring device,

Fig. 7 illustrates schematically and exemplarily an arrangement of the pressure sensing pad on the shell,

Fig. 8 shows schematically and exemplarily a pressure transducer housing and its connection to a fluid tube and an electrical cable,

Fig. 9 shows schematically and exemplarily the pressure sensing pad being connected to the pressure transducer via the tube and the arrangement of a vent valve on a further tube extending from the pressure transducer housing,

Fig. 10 shows schematically and exemplarily a 3D mesh before being arranged within the pressure sensing pad,

Fig. 11 shows schematically and exemplarily the pressure sensing pad including the 3D mesh in a cross section,

Fig. 12 shows schematically and exemplarily an embodiment with a pressure transducer being arranged within the pressure sensing pad,

Fig. 13 shows schematically and exemplary a measured tissue pressure and further values derived from the tissue pressure measurement,

Figs. 14 to 17 illustrate calculations of different features for a pressure pulse of the measured tissue pressure,

Figs. 18A to 21B show exemplarily, actual tissue pressure measurements achieved with a liquid-filled pressure sensing pad and an air-filled pressure sensing pad in comparison to each other as well as to a conventional oscillometric measurement, and

Fig. 22 shows a flowchart exemplary illustrating an embodiment of a method for determining a physiological parameter of a subject.

## DETAILED DESCRIPTION OF EMBODIMENTS

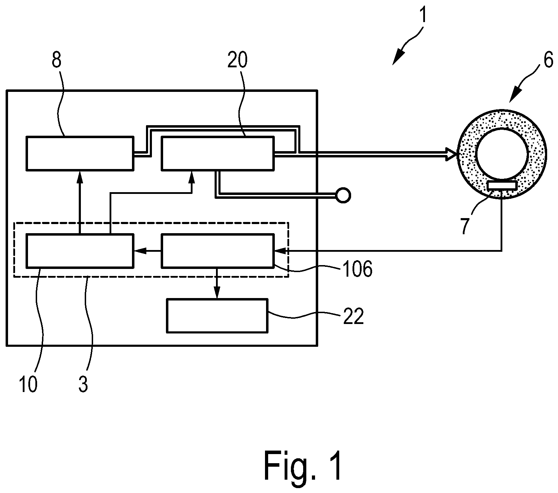

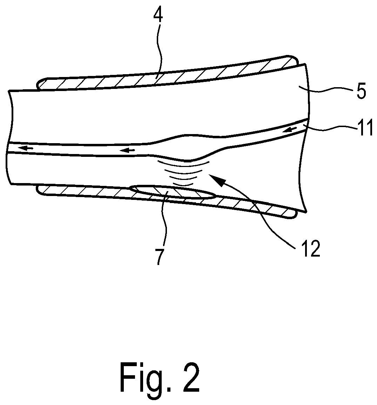

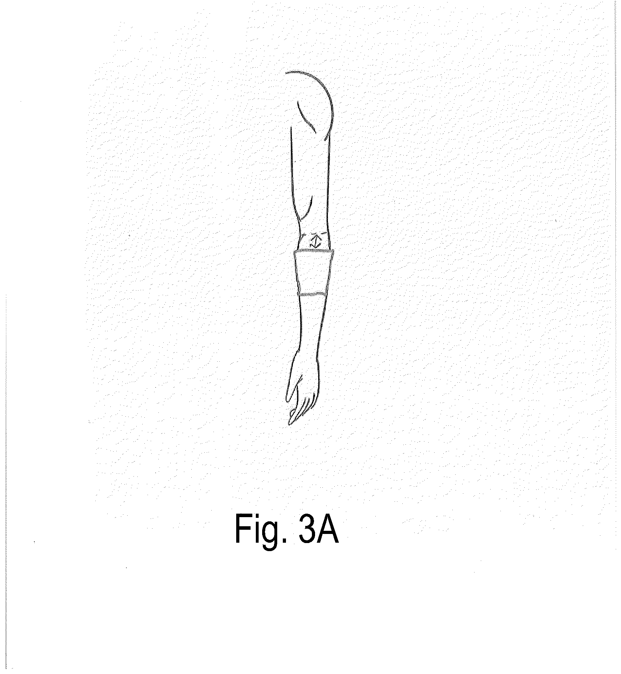

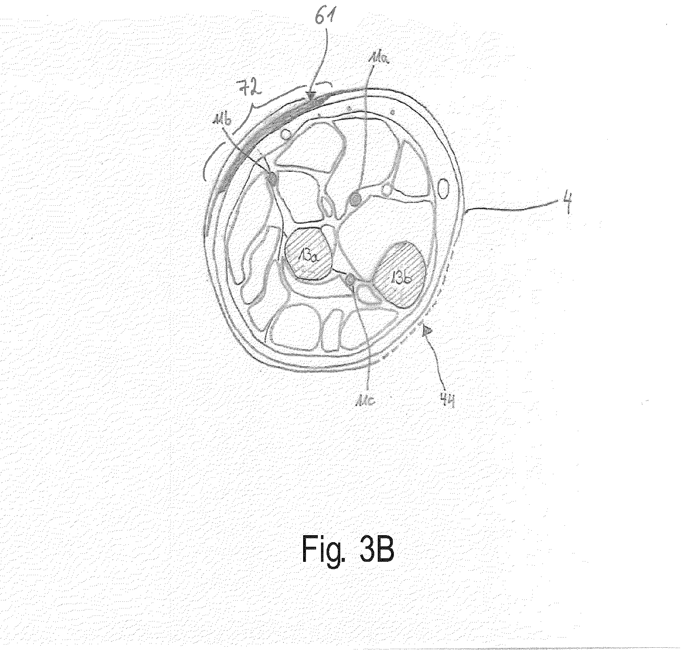

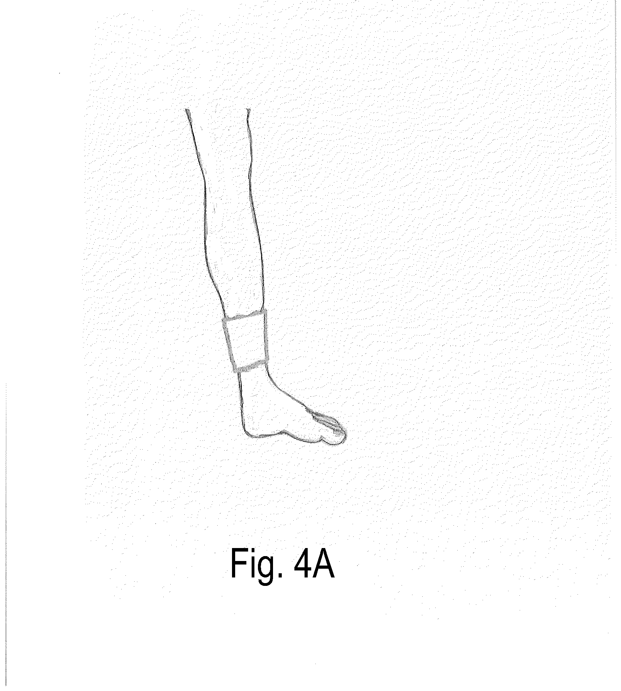

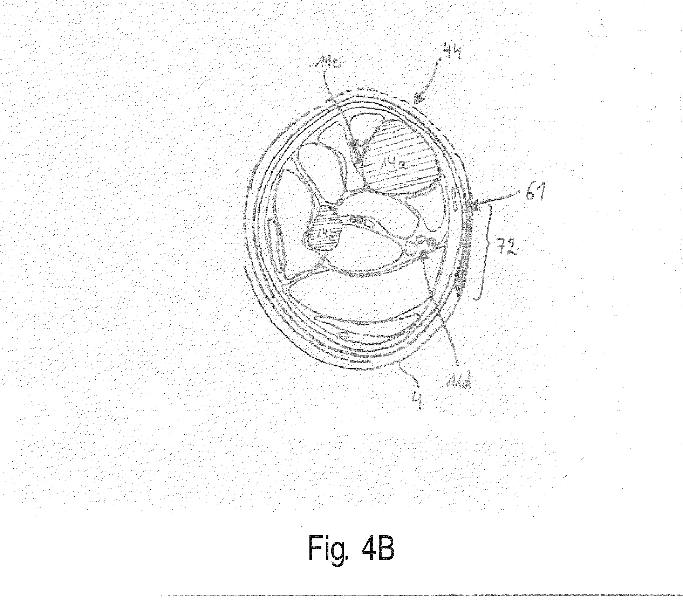

Fig. 1 shows schematically and exemplarily an apparatus 1 for determining a physiological parameter of a subject like the blood pressure. The apparatus 1 comprises a pressure measuring device 6 with a shell 4 which can be seen in Fig. 2 as well as in Fig. 3B and 4B, and which is configured to encase a part 5 of the subject, through which blood flows. The body part 5 of the subject may be an arm of the subject, wherein in Fig. 2 within the arm an artery 11 is shown and wherein the arrows within the artery 11 indicate the flow direction of the blood away from the heart. The part 5 may generally be an upper arm, in which case the artery 11 could be a brachial artery, but it may be preferred that it is a proximal part of a forearm as indicated Fig. 3A and Fig. 3B, or a distal part of a lower leg as indicated Fig. 4A and Fig. 4B. The pressure measuring device 6 further comprises a pressure sensing device of which a part 7 in the form of a fluid-filled pressure sensing pad 61 as described in more detail below, is arranged on the inner side of the shell 4 and which is configured to measure the pressure on the outer skin of the encased body part 5 of the subject. The measured pressure can also be regarded as being tissue pressure (TP). As indicated in Fig. 2, a pulse wave within the brachial artery 11 causes a pressure wave 12 which is transmitted to the air-filled pressure sensing part 7, i.e. the pressure sensing pad 61, of the pressure sensing device via the tissue of the body part 5. The shell 4 is not shown in Fig. 1 for clarity reasons. The shell 4 preferentially is a kinking-proof shell as described above.

While not shown in Fig. 2, the pressure measuring device 6 further comprises a pressure application element being a pressure cuff comprising a fluid bag which encloses the shell 4 and which is inflatable by using a pump 8 for applying, from outside the shell 4, pressure to the shell 4 and thereby to the encased body part 5 of the subject.

The apparatus 1 further comprises a processor 3 which is configured to control the pump 8 such that the pressure application element increases the applied pressure in a measurement time period extending till an end measurement time point and decreases the applied pressure in a following post-physiological-parameter-measurement time period and that the pressure sensing device with the pressure sensing part 7 corresponding to the pressure sensing pad 61, measures the pressure on the skin, i.e. the tissue pressure TP, at least during the measurement time period. Moreover, the processor 3 is configured to control the pump 8 such that it increases the applied pressure - via the cuff - with a first rate in a pre-measurement time period which is followed by the measurement time period in which the applied pressure is increased with a second rate, wherein the first rate is larger than the second rate. The control of the measurement device such that the cuff is inflated and hence the applied pressure is increased is illustrated in Fig. 1, i.e. the bold arrows indicate the inflation situation.

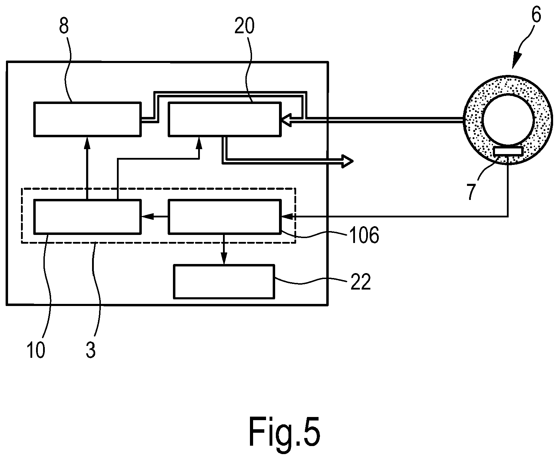

The apparatus 1 comprises a valve 20 which, when the valve 20 is opened, allows the compressed air within the apparatus 1 to leave the apparatus into the surrounding atmosphere for deflating the cuff. In Fig. 5 this deflation situation, in which the pump 8 is switched off, is indicated by the bold arrows.

In the pre-measurement time period, which could also be regarded as being a fast inflation period, the processor 3 controls the apparatus 1 such that the valve 20 is closed and the pump 8 inflates the pressure cuff with the larger first rate. In the following measurement time period the processor 3 also controls the apparatus 1 such that the valve 20 is closed, but the pump 8 is controlled such that the inflation of the cuff 6 is continued with the lower second rate. The measurement time period could therefore also be regarded as being a slow inflation period.

The processor 3 can be regarded as comprising a controlling part 10 for controlling the pump 8 and the valve 20 and a processing part 106, which especially is configured to carry out some calculations that will be explained further below. The apparatus 1 can also comprise a display 22 for displaying the determined physiological parameter like the blood pressure.

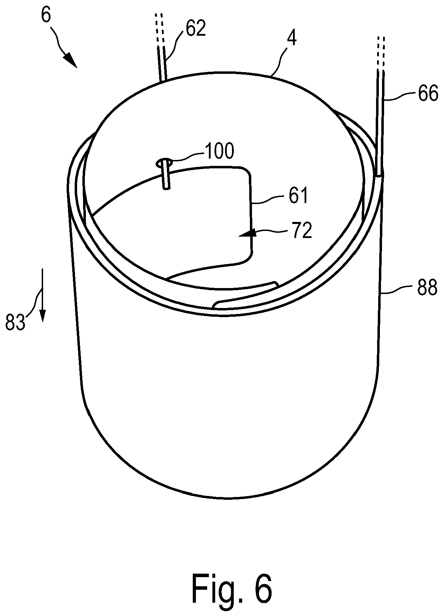

Fig. 6 shows schematically and exemplarily the pressure measuring device 6. The pressure application element, i.e. the pressure cuff, in this case substantially corresponds to a fluid bag 88 that is fillable with fluid for applying the pressure to the body part 5 via the shell 4. For filling the fluid bag 88 with the fluid and for releasing the fluid from the fluid bag 88, the fluid bag 88 can be connected to the pump 8 and the valve 20 via a tube 66. In Fig. 6, the shell 4 is arranged axially aligned inside of the fluid bag 88, i.e. the fluid bag 88 could be viewed as a hollow sleeve around the shell 4.

The shell 4, which is preferably made from a plastic material so as to be semi-rigid, may be viewed as having the shape of a hollow cylinder or cone whose bottom and top has been removed and which has two overlapping ends in circumferential direction. The cylinder (or cone) axis corresponds to the proximal-distal direction 83. While not specifically indicated in Fig. 6, an angle by which the surface of the shell 4 is tapered from a proximal side towards a distal side of the shell 4, which may also be viewed as a cone angle of the shell 4, is preferentially between 4° and 5°, particularly 4.5°. The angle may be measured, in a plane including a central axis of the shell 4, as an angle between a) a line of intersection between an outer surface of the shell 4 and the plane, and b) a line in the plane being parallel to the central axis, intersecting the line of intersection between the outer surface of the shell 4 and the plane.

When the fluid bag 88 is being filled with fluid, its volume increases, which leads to a decreased inner diameter of the fluid bag 88 and hence, by increasingly overlapping ends of the shell 4 in circumferential direction, also a decreased diameter of the shell 4. When the shell 4 and the fluid bag 88 encase a body part of a subject, the decreased diameters translate to an increased pressure on the body part.

While in this case the fluid bag 88 is closed in circumferential direction, such that it would be slid axially over the shell 4 for applying pressure, generally the pressure application element could also be open in circumferential direction, i.e. similarly as the shell 4. In that case, it may be wrapped around the shell 4, whereafter it may be closed in circumferential direction by, for instance, a hook-and-loop or a button mechanism.

Fig. 3A schematically and exemplarily shows a preferred measurement position for subjects being severely obese. Due to the generally large upper arm volumes in these subjects, it may be preferred to instead use the forearm, particularly a proximal part of the forearm, for measurements with the pressure measuring device 6. The pressure measuring device 6 may in particular be positioned on the forearm such that its proximal outer edge has a predefined distance to a predefined point where the upper arm meets the forearm.

Fig. 3B shows schematically and exemplarily a cross-section through the forearm and the shell 4 of the pressure measuring device 6 being applied around the forearm, wherein the cross-section is taken in a plane perpendicular to the proximal-distal direction 83. From the pressure measuring device 6 surrounding the forearm, only the shell 4 and the pressure sensing pad 61 being arranged in a pressure sensing area 72 of an inner surface of the shell 4 is shown in Fig. 3B. Furthermore, the ulnar artery 11a, the radial artery 11b and the posterior interosseous artery 11c are indicated, as well as the radial shaft bone 13a and the ulnar shaft bone 13b. The remaining anatomical structures sketched in Fig. 3B include muscles, veins and nerves. In Fig. 3B, left corresponds anatomically to the lateral direction, right corresponds anatomically to the medial direction, up corresponds anatomically to the anterior direction, and down corresponds anatomically to the posterior direction.

Fig. 4A schematically and exemplarily shows an alternative measurement position that may also be preferred for subjects being severely obese. As illustrated, according to this alternative the body part used for the measurement with the pressure measuring device 6 is a distal part of a lower leg. The pressure measuring device 6 may again be positioned on a predefined position relative to an anatomical reference point, which in this case may be on the ankle, for instance.

Fig. 4B shows schematically and exemplarily a cross-section through the distal lower leg and the shell 4 of the pressure measuring device 6 being applied around the distal lower leg, wherein the cross-section is taken in a plane perpendicular to the proximal-distal direction. From the pressure measuring device 6 surrounding the forearm, only the shell 4 and the pressure sensing pad being arranged in the pressure sensing area 72 of an inner surface of the shell 4 is shown in Fig. 4B. Furthermore, the anterior tibial artery 11c and the posterior tibial artery 11d are indicated, as well as the tibia bone 14a and the fibula bone 14b. The remaining anatomical structures sketched in Fig. 4B include muscles, veins and nerves. In Fig. 4B, left corresponds anatomically to the lateral direction, right corresponds anatomically to the medial direction, up corresponds anatomically to the anterior direction, and down corresponds anatomically to the posterior direction.

Fig. 6 also shows the pressure sensing pad 61 being arranged on an inner surface of the shell 4 in a pressure sensing area 72. The pressure sensing area 72 corresponds to an area in which tissue pressure pulses can be sensed and therefore could be regarded as an active sensing area of the pressure sensing pad 61. When the pressure measuring device 6 is applied such that it surrounds a body part of the subject, the shell 4 and hence also the pressure sensing pad 61 will extend a) along the body part in proximal-distal direction 83 and b) around the body part in the circumferential direction.

The pressure sensing area 72 and thus the pressure sensing pad 61 covers a mid-portion of the inner surface of the shell 4 as viewed in proximal-distal direction. For guiding pressure from the pressure sensing pad 61 to the pressure transducer, a tubing or tube 62 appropriate for invasive blood pressure measurement is provided, which pierces the shell 4 through a hole 100. On the exterior of the shell 4, a portion of the tube 62 can be arranged in between the shell 4 and the fluid bag 88.

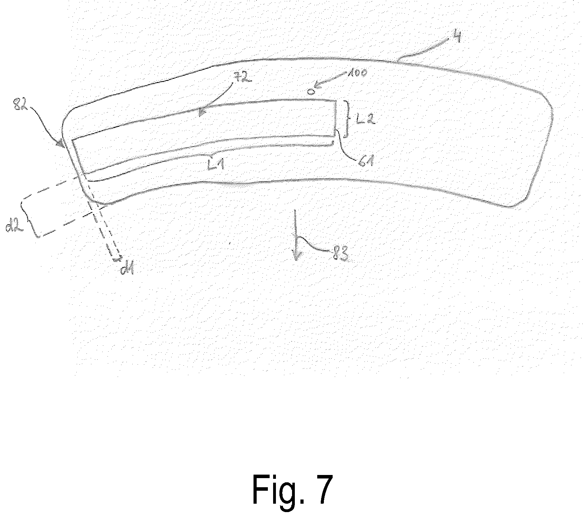

Fig. 7 shows schematically and exemplarily the shell 4 in a flat condition, including the pressure sensing pad 61 with its pressure sensing area 72 and the hole 100. Regarding the positioning and the dimensions of the pressure sensing pad 61, it can be identified with the pressure sensing area 72.

In the flat condition of the shell 4 and hence also of the pressure sensing area 72 shown in Fig. 7, the pressure sensing area 72 essentially has the shape of a rectangle that is prolonged and slightly curved in the lateral direction. The curvature of the rectangle may generally follow a curvature of an outer proximal and/or an outer distal edge of the shell 4, wherein it may be slightly smaller. The side lengths of the pressure sensing area 72 will, like the dimensions of the shell 4, be manufactured depending on the intended subject and/or body part. Fig. 7, for instance, shows a shell 4 configured to surround an upper arm, a forearm or a lower leg of a severely obese adult. In this case, the shell 4 may have a dimension between 80 mm and 100 mm in the proximal-distal direction 83 and a dimension of at least 260mm, preferably at least 360 mm, in the circumferential direction, while the pressure sensing area 72 has a side length L1 that is preferably between 70 mm and 215 mm in the lateral or circumferential direction and a side length L2 between 25 mm and 45 mm, particularly between 30 mm and 40 mm, in the proximal-distal direction 83. The side length L1 may preferably be 45% and 95%, more preferably between 59% and 83%, of an inner circumference of the shell 4 in the circumferential direction, the latter optionally corresponding to a maximum working range of an body part circumference for which the shell 4 is made.

It has been recognized that, the longer the pressure sensing area 72 is in lateral direction, i.e. the larger L1, the less grave and/or likely are measurements errors to occur due to misplacements of the shell 4 on the respective body part. For instance, the shell 4 may comprise an arterial shell positioning line indicated by a visible marking (not shown in Fig. 7) in order to assist a user to place the shell 4 on a subject's body part, the aim being to apply the shell 4 such that the arterial shell positioning line is aligned with a respective artery in the body part. In case of misalignment, measurement errors may be expected, which could also be referred to as rotation errors. These errors can be mitigated with a laterally prolonged shape of the pressure sensing area 72, as already explained further above. However, as indicated in Fig. 3B and 4B, smaller circumferential extents of the pressure sensing area 72 are also possible and may particularly not lead to a decrease in measurement accuracy if the shell 4 is placed accurately on the respective body part.

With a laterally prolonged shape as indicated in Fig. 7, the pressure sensing area 72 hence extends into an end portion of the shell 4. As shown in Fig. 7, the end portion is preferentially tapered with respect to the remaining part of the shell 4. In the illustrated embodiment, the lateral edge of the pressure sensing area 72 that extends into the end portion has a distance d1 to the lateral edge 82 of the shell 4 that is at least 5 mm, as measured in the lateral direction, i.e. the direction perpendicular to the proximal-distal direction 83.

The pressure sensing area 72 extends along a middle of the shell 4. That is to say, the pressure sensing area 72 extends along a virtual midline of the shell 4, the virtual midline marking a mid-height of the shell, i.e., a middle of the shell in proximal-distal direction 83. In particular, Fig. 7 illustrates the preferred case that the pressure sensing area 72 covers the middle of the shell 4, i.e., the virtual midline, along a whole circumferential extent of the pressure sensing area 72. This has been found advantageous for measurements on obese patients, particularly when the forearm or a lower leg is used as body part 5 for the measurement.

The distance d2 between an outer distal edge of the pressure sensing area 72 and an outer distal edge of the shell 4 is within a range from 20% to 40% of the entire height of the shell 4 from its outer distal edge to its outer proximal edge. In a preferred embodiment, the distance d2 between the outer distal edge of the pressure sensing area 72 and the outer distal edge of the shell 4 is 35%. The distance d2 may be measured in the proximal-distal direction or in the direction of the lateral edge of the pressure sensing area 72. Moreover, the distance d2 may be measured from the corner of the pressure sensing area 72 where the lateral edge and the outer distal edge of the pressure sensing area 72 meet. It should be noted that the values, or percentages, given above for d2 may particularly apply in the case that the end portion into which the pressure sensing area 72 extends is tapered. In that case, the outer distal edge of the shell 4 with respect to which the distance d2 is measured may thus be tapered.

Preferably, the pressure sensing area 72 is, in proximal-distal direction, centered around the middle of the shell 4. In other words, a proximal edge and a distal edge of the pressure sensing area 72 preferably have a same distance to a virtual midline of the shell 4 in proximal-distal direction 83. Accordingly, a distance between the proximal edge of the pressure sensing area 72 and a proximal edge of the shell 4 is preferentially equal to the distance d2 between the outer distal edge of the pressure sensing area 72 and the outer distal edge of the shell 4. This equality may only be an approximate equality if the proximal edge of the shell 4 is not tapered or tapered differently than the distal edge of the shell 4 towards the end portion of the shell 4 into which the pressure sensing area 72 extends.

It has been found that the above described arrangement and dimensions of the pressure sensing area can lead to an improved pressure signal transmission from the body part of the subject to the pressure sensing pad 61. With the preferred conical shape of the pressure measuring device 6 and particularly of the shell 4 corresponding to the conical shape of a body part 5 to be surrounded by the shell 4, the occlusion of the respective artery happens in the middle of the shell as viewed in proximal-distal direction, wherein for this reason it is preferred that the location of the pressure sensing area 72 is at mid-height between the distal edge of the shell 4 and the proximal edge of the shell 4. Meanwhile, placing the pressure sensing area 72 at mid-height of the shell 4 can also be advantageous for non-conical shells 4, particularly when body part used for the measurement is non-conical. Furthermore, the lateral prolongation of the pressure sensing area extending towards an end portion, i.e. towards a lateral edge 82, of the shell allows to cover a relatively large portion of the respective body part, even if the body part is relatively large itself, wherein the distance of the pressure sensing area 72 to the edge 82 avoids mechanical contact to the pressure sensing area 72 during application, while still allowing for a smooth sliding function of the edge 82.

It should be noted that the preferred slightly conical shape of the shell 4 can already be appreciated from the curved shape of the shell 4 in its hypothetical uncurled, planar state shown in Fig. 7. Moreover, while not particularly emphasized in Fig. 7, the conicity of the shell 4 may be identified from a length of the proximal edge of the shell 4 being larger than a length of the distal edge of the shell 4. The difference in length may be such that, as indicated further above with reference to Fig. 6, a cone angle between 4° and 5°, preferably of 4.5°, results, which has particularly been found to conform well to both forearm and lower leg shapes of obese patients.

It should also be noted that Figs. 6 and 7 do not further illustrate any segmentation of the shell 4 in circumferential direction regarding a flexibility and/or thickness, although this may be preferred to allow for a more uniform pressure application to body parts such as the forearm or a lower leg with prominent bones. However, this is shown in both Fig. 3B and 4B, where a respective shell segment 44 being thinner and/or more flexible than a remaining portion of the shell 4 is indicated by a dashed section of the line indicating the shell 4. As shown, the shell segment 44 may be positioned in circumferential direction so as to be placed on an ulnar bone 13b or a tibia bone 14a.

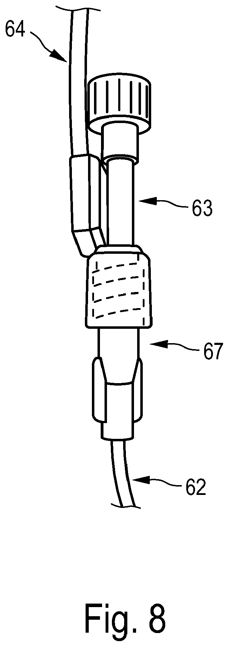

Fig. 8 shows schematically and exemplarily a pressure transducer housing 63, which is connected with the pressure sensing pad 61 via the tube 62 as shown in Fig. 6. While not seen in Fig. 8, a pressure transducer is arranged in the pressure transducer housing 63. The pressure transducer preferentially is a differential pressure transducer (DPT). While it may be preferred that the tube 62 enters the pressure transducer housing 63 smoothly without a dedicated connecting element, in this case the tube 62 is connected to the pressure transducer housing 63 via a connector 67 being, for instance, a Luer connector. The measured pressure signal is guided to the controller 3 via a cable 64.

The pressure sensing pad 61, the tube 62 and the pressure transducer housing 63 may in principle be filled with liquid, in order to provide a hydraulic coupling between the air-pressure sensing pad 61 and the pressure transducer. However, it has been found that not only any liquid in the pressure sensing pad 61 is dispensable, but also any liquid in the tube 62 and the pressure transducer housing 63. For the subsequent description, an air-filled pressure sensing pad 61 shall therefore be assumed, and also that the tube 62 and the pressure transducer housing 63 are filled with air, leading to the above indicated advantages regarding production costs and zeroing of the pressure transducer. In a particular variant without substantial drawbacks being likely, the pressure transducer housing 63 may still be filled with liquid, such as glycerol.

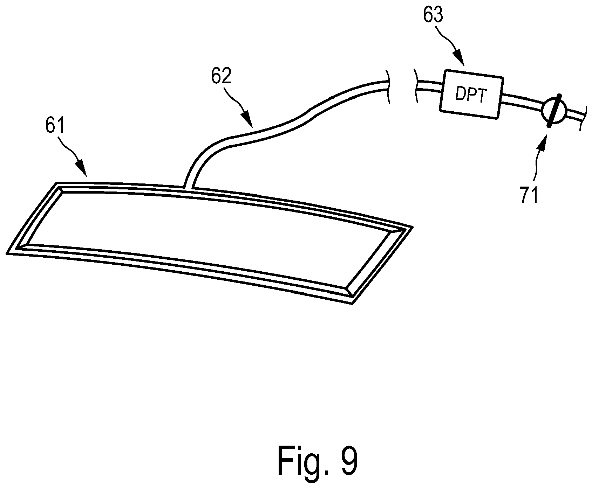

Fig. 9 shows schematically and exemplarily the pressure sensing device of the pressure measuring system 6. The pressure sensing device includes the pressure sensing pad 61, the pressure transducer being arranged in a housing 63 as described above, and the tube 62 connecting the pressure sensing pad 61 with the pressure transducer housing 63 and hence the pressure transducer. In the embodiment illustrated by Fig. 9, a further tube part extends from the pressure transducer housing 63, on which a valve 71 for venting the pressure sensing device is arranged. The further tube part can be a further part of the tube 62, which would in this case extend through the pressure transducer housing 63, or it could be a part of a further tube different from the tube 62. Moreover, the valve may also be arranged in the part of the tube 62 extending from the pressure sensing pad 61 to the pressure transducer housing 63, in which case no further tube part at all may further extend from the pressure transducer housing 63.

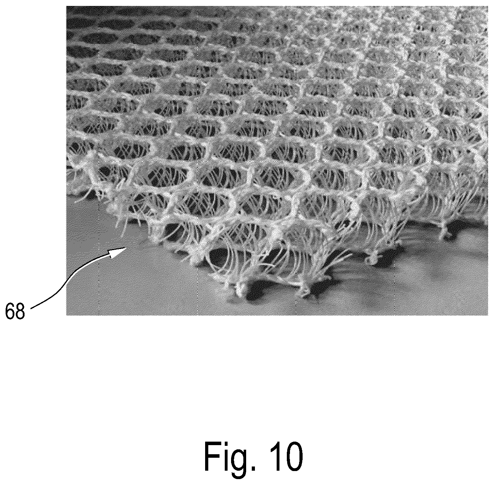

In order to keep the air in the pressure sensing pad 61 at a minimum air thickness throughout the pad, it is preferred that a mesh 68 is arranged within the pressure sensing pad 61 as schematically and exemplarily illustrated in Fig. 10. Preferentially, the mesh 68 is a three-dimensional mesh. The three-dimensional mesh 68 generates air channels within the pressure sensing pad 61 and it can reduce the likelihood of a closure of the end of the tube 62 that is arranged within the pressure sensing pad 61. Moreover, the three-dimensional mesh 68 can reduce the likelihood of forming larger non-air filled compartments within the pressure sensing pad 61.

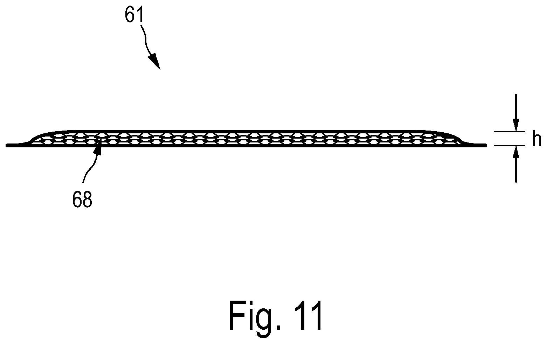

As illustrated schematically and exemplarily by Fig. 11 in terms of a cross-section through the pressure sensing pad 61, the air in the pressure sensing pad 61, i.e. the air bed or cushion, being supported by the mesh 68, has an air thickness h which, in a flat condition of the pressure sensing pad 61, is within a range from 0.60 mm to 1.10 mm. Preferentially, the air thickness is 0.79 mm in the flat condition of the pressure sensing pad 61. The air thickness is preferably measured as the thickness of the fluid between the foils or walls of the pressure sensing pad 61, when the pressure sensing pad is in a flat condition. In use, the foils or walls, which are relevant here, are the foils or walls being parallel to the inner surface of the shell 4.

It has been found that the above air thickness range from 0.60 mm to 1.10 mm can prevent a pressure offset in a mounted shape at a relatively small circumference and that it can also minimize wrinkles or the likelihood of separation in air compartments over the area of the pressure sensing pad 61, wherein particularly the value of 0.79 mm has been found to be optimal for different sizes of the pressure measuring device 6. In this way, an air thickness within the range from 0.60 mm to 1.10 mm, especially of 0.79 mm, can lead to an improved measurement performance. In particular the measurement performance can be maintained throughout a working range of the pressure measuring device 6. The working range may be defined by a minimum and a maximum circumference of the respective body part and hence the pressure measuring device 6 for which a measurement should be carried out. For instance, a pressure measuring device 6 intended for blood pressure measurements on forearms of severely obese adults, the working range may be from 26 cm to at least 36 cm forearm circumference.

Generally, under- or overfilling with air can influence the measurement performance, for instance, can lead to a poor signal amplitude or to a pressure offset due to an increasing internal pressure. For this reason, the valve 71 can be used for venting the pressure sensing device, in order to equalize the internal pressure with the atmosphere. The valve, which may be a stopcock, for instance, may be operable by the controller 3. In particular, the controller may be configured to close the valve 71 for zeroing the pressure transducer when the cuff is not applied to the body part. In this way, the sensed pressure signals can indicate pressure relative to atmospheric pressure.

Hence, the mesh 68 and the valve 71 cooperate in helping to keep the height of the pressure sensing pad 61 at the right level. Regarding the physical properties of the mesh 68, it could be said that the mesh 68 is preferably tough enough to reproducible and endurably raise the height of the pressure sensing pad 61 in the non-attached pressure measuring device 6 when open to the atmosphere (such as, for instance, by virtue of an opened valve 71), but soft enough not to additionally dampen the pressure signal, i.e., the amplitude of the pressure pulses from the respective body part.

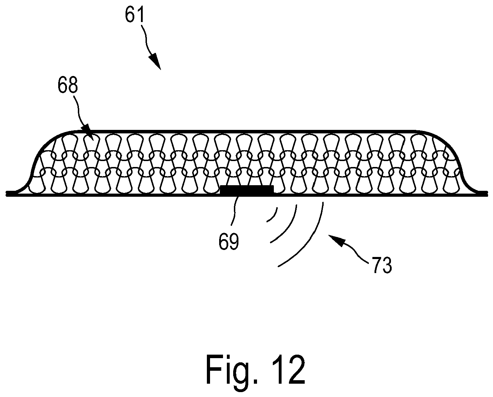

Fig. 12 shows schematically and exemplarily an embodiment in which a pressure transducer 69 is arranged within the pressure sensing pad 61. In this embodiment, no tube 62 connecting the pressure sensing pad 61 to an external pressure transducer 63 and also no cable 64 for transmitting electrical signals from the pressure transducer 69 to a processor are needed (although such an "external" pressure transduction may still be maintained as a reference or in order to allow for a redundant measurement). Instead, the pressure transducer 69 acquires the pressure signals directly inside the pressure sensing pad 61 and transmits them in terms of corresponding wireless signals 73 to a processor. This construction can lead to a further increased accuracy of measuring the pressure from the body part 5, because the propagation path of the pressure signals is substantially shortened and also generally possible kinks in the tube, mechanical noise generated therein by touching or air bubbles in the tube cannot adversely affect the pressure measurement.

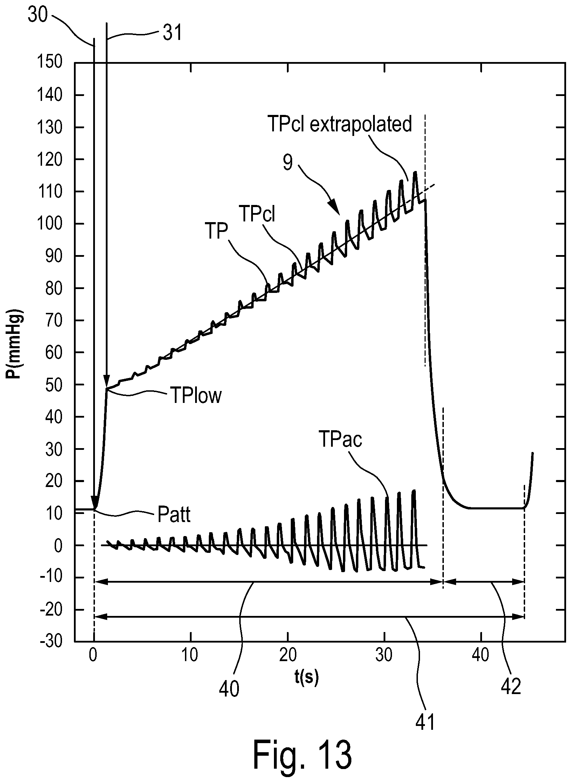

Fig. 13 schematically and exemplarily illustrates the measured pressure TP versus time t. The first inflation in the pre-measurement time period starts with a tissue pressure TP being an attachment pressure Patt that is the measured tissue pressure in the uninflated cuff 60. This attachment pressure Patt can range, for instance, from 0 to 15 mmHg. An attachment pressure Patt up to 15 mmHg has turned out to not result in venous congestion for more than 12 hours, thereby making the pressure measuring device 6 optimally suitable for longer term monitoring. For this reason, it is preferred that the attachment pressure Patt is not larger than 15 mmHg. In Fig. 13 the arrow 30 indicates the start of the pre-measurement time period, i.e. the start of the fast inflation period. During this pre-measurement time period the part of the overall tissue pressure range, which has no or substantially no information for the determination of the blood pressure, shall be passed as fast as possible. Therefore, the inflation rate is preferentially as large as possible during the pre-measurement time period. For instance, the inflation rate with respect to the tissue pressure TP can be equal to or larger than 8 mmHg/s. This pre-measurement time period with a fast inflation rate ends at a tissue pressure value which is indicated in Fig. 13 by "TPlow".

The tissue pressure value TPlow also indicates the start of the measurement time period with the slower second inflation rate. In Fig. 13 this start of the measurement time period, which can also be regarded as being a slow inflation period, is indicated by the arrow 31.

In Fig. 13 the time interval 40 indicates the inflation-deflation time period from the start 30 of fast inflation until the tissue pressure TP has dropped below 20 mmHg during the fast deflation, in order to allow venous return. The time period 41 indicates a cycle time being the time between a start of a measurement and a start of a following measurement and the time period 42 indicates a break period being the difference between the inflation-deflation time period 40 and the cycle time 41.

Fig. 13 also illustrates mean pressure TPcl that can be regarded as being a tissue clamping pressure affecting the tissue when the pressure measuring device 6 is attached to the arm 5, for instance, to the upper arm, of the subject. The mean pressure TPcl can be calculated by applying a low pass filter to the tissue pressure TP, wherein the low pass filter might be located within the processor 3. The processor 3 is preferably configured to determine an alternating component TPac of the tissue pressure by subtracting the mean pressure TPcl from the measured tissue pressure TP, i.e. TPac = TP - TPcl. In Fig. 13 the TPac curve is shown enlarged by a factor of 2, in order to improve visibility of this curve.

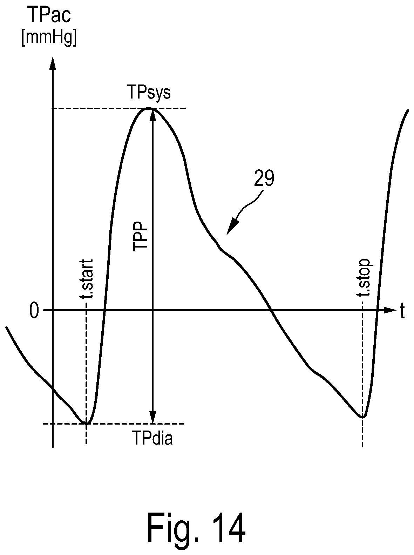

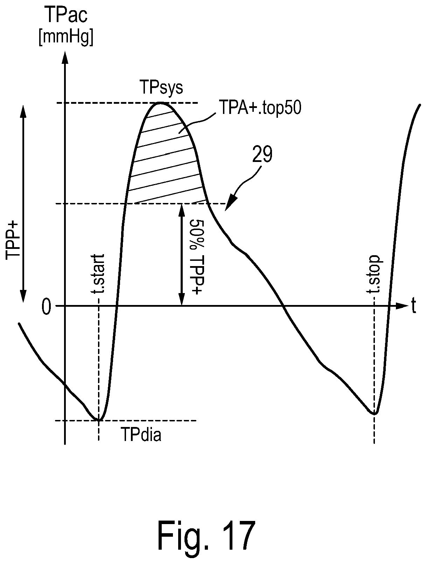

During the slow inflation period, i.e. during the measurement time period, TP pulse curves and/or TPac pulse curves are recorded, which may be analyzed simultaneously, i.e. online, wherein the TP pulse curves and/or the TPac pulse curves have tissue pressure waveforms (TPW) comprising information which allows for an accurate determination of the respiration rate. Since the blood pressure is noninvasively measured (although it nevertheless has an accuracy which is comparable to the accuracy of invasively measured blood pressure), it can be abbreviated with niBP meaning "noninvasive blood pressure". A TPac pulse curve is schematically and exemplarily shown in Figs. 14 to 17.

In the following the TPac pulse curves are used for determining features for the pressure pulses 9. The TPac pulse curves can therefore be regarded as being feature determination pulse curves or feature determination pulses. Figs. 14 to 17 hence show feature determination pulse curves or feature determination pulses 29 being TPac pulses. In another embodiment the feature determination pulses can also directly be the TP pulse curves, i.e. the pressure pulses 9 can be directly used for determining the features. The processor 3 is configured to determine for a respective pressure pulse of the plurality of pressure pulses 9 at least one feature which characterizes the respective pressure pulse.

In an embodiment, the processor 3 is configured to determine a difference TPP between a maximum measured pressure and a minimum measured pressure of the respective feature determination pulse 29 as a feature for the respective pressure pulse. This feature will be described with reference to Fig. 14 in the following.

The difference TPP is a difference of a feature determination pulse's maximum systolic pressure (TPsys) and the feature determination pulse's pressure at an end-diastolic point (TPdia), which is preferably the minimum pressure of the respective feature determination pulse. In Figs. 14 to 17 the terms "t.start" and "t.stop" indicate the start and the end, respectively, of the respective pulse 29.

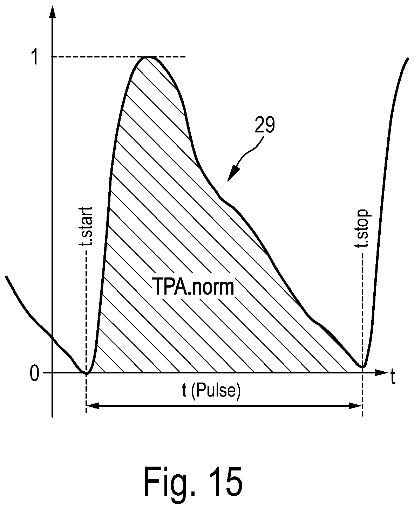

The processor 3 can also be configured to determine the pulse duration (t(Pulse)) being the time difference between an end-diastolic point and a following end-diastolic point of the feature determination pulse 29. This feature can also be defined as being the temporal difference between the start of the respective pulse (t.start) and the end of the respective pulse (t.stop). This feature is indicated in Fig. 15.

The processor 3 can also be adapted to determine the pulse area (TPA) of the respective pulse 29 being the area under the respective pulse curve within the time defined by t.start to t.stop and ranging from the feature determination pulse's pressure at an end-diastolic point (TPdia) to a feature determination pulse's maximum systolic pressure (TPsys). Preferentially, the pulse area TPA is scaled to TPP=1 as indicated in Fig. 15. This scaled pressure pulse area is named "TPA.norm".

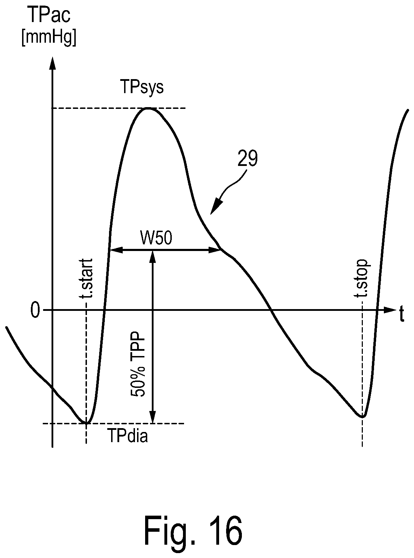

The processor 3 can also be adapted to determine the pulse width at half maximum (W50) of the respective pulse 29 as indicated in Fig. 16.

Moreover, the processor 3 can be adapted to determine the area TPA+.top50 enclosed by an upper part of the feature determination pulse 29, as schematically illustrated in Fig. 13. The upper end of the area TPA+.top50 enclosed by the upper part is at the feature determination pulse's 29 maximum systolic pressure TPsys and the lower end of the area TPA+.top50 of the upper part is between the feature determination pulse's 29 maximum systolic pressure TPsys and a pressure value which corresponds to the mean TPcl of the measured pressure TP. Since the feature determination pulse 29 has been determined by subtracting the mean TPcl from the measured pressure TP, the pressure value, which corresponds to the mean TPcl of the measured pressure TP, is zero. Preferentially, the processor 3 is configured to determine the area TPA+.top50 enclosed by the upper part of the feature determination pulse such that the lower end of the upper part is at a pressure value which corresponds to half of the pressure distance TPP+ between the feature determination pulse's maximum systolic pressure TPsys and the pressure value which corresponds to the mean TPcl of the measured pressure TP.

Figs. 14 to 17 show a TPac pulse curve such that in this example also the features have been defined based on the TPac pulse curve. However, as mentioned above, it is also possible to define these or other features based on a TP pulse curve.

The processor 3 is further configured to determine for a respective pressure pulse a blood pressure determination value TPWP_M based on at least one determined feature such that for several pressure pulses, which are present at different times, several blood pressure determination values TPWP_M are determined, wherein the several blood pressure determination values TPWP_M determined for the several pressure pulses and hence for several times form a blood pressure determination curve TPW_M-curve. The processor 3 is configured to determine the blood pressure based on the blood pressure determination curve TPW_M-curve, for instance as explained in WO 2018/210931 A1. In particular, the processor 3 is configured to determine a position of a maximum (TPW_M-curve.max) of the blood pressure determination curve TPW_M-curve and to determine the blood pressure based on the determined position and the measured pressure TP. For instance, the processor 3 is configured to determine the systolic arterial blood pressure (SAPni) based on the mean TPcl of the measured pressure TP at the determined position of the maximum (TPW_M-curve.max) in accordance with following equation:

$$SAPni = \alpha \cdot (TPcl@TPW\_M\text{-}curve.max) \qquad , \qquad (1)$$

wherein the parameter $\alpha$ can be predetermined by calibration.

Preferentially the processor is configured to provide a function which receives as input the at least one feature of a respective pressure pulse and which outputs a respective blood pressure determination value which forms, together with the blood pressure determination values determined for the other pressure pulses, the blood pressure determination curve. The function has at least one parameter which can be determined by calibration, wherein by invasive means reference blood pressure values are determined very accurately and the at least one parameter is determined such that the apparatus yields the very accurately invasively measured blood pressure values with high statistical precision and accuracy.

In a preferred embodiment the processor 3 is configured to form the blood pressure determination curve TPW _M-curve based on blood pressure determination values TPWP_M which depend only on the feature being the difference TPP between the feature determination pulse's maximum systolic pressure TPsys and the feature determination pulse's pressure at the end-diastolic point TPdia. Thus, in an embodiment the function, which is used for determining the blood pressure determination values TPWP_M, depends on TPP, but not on any other features of the pressure pulses. In particular, the blood pressure determination values TPWP_M can be determined in accordance with following equation:

$$TPWP\_M = d1 \cdot TPP^{exp1}, \qquad (2)$$

wherein d1 and exp1 are predetermined constants. For instance, d1 and exp1 can be predetermined by calibration. However, it has been found that they can also both be chosen to be equal to one, i.e. d1 =1=exp1, such that TPWP_M = TPP.

Moreover, in a preferred embodiment the processor 3 is configured to form the blood pressure determination curve TPW _M-curve based on blood pressure determination values TPWP_M which depend only on a) the feature being the difference TPP between the feature determination pulse's maximum systolic pressure TPsys and the feature determination pulse's pressure at the end-diastolic point TPdia and b) the feature being the area TPA+.top50 enclosed by the upper part of the feature determination pulse. Thus, in an embodiment the function, which is used for determining the blood pressure determination values TPWP_M, depends on TPP and TPA+.top50, but not on further features of the pressure pulses. In particular, the blood pressure determination values TPWP_M can be determined in accordance with following equation:

$$TPWP\_M = d2 \cdot TPA+.top50^{exp2} \cdot TPP^{exp3}, \qquad (3)$$

wherein d2, exp2 and exp3 are predetermined constants. For instance, d2 can be equal to one, i.e. d2 = 1, wherein exp2 and exp3 can be predetermined by calibration. However, it is also possible that the constant d2 is determined by calibration. In a preferred embodiment, d2 and exp3 are both equal to one (d2=1, exp3=1) and exp2 is equal to 0.7 (exp2=0.7).

In an embodiment the blood pressure determination curve TPW _M-curve can be a smoothed curve such that the maximum of the blood pressure determination curve is also smoothed. The smoothing procedure used for smoothing the blood pressure determination curve can include, for instance, filtering and/or fitting. Further, several pressure pulses can be considered, after the smoothed maximum, to detect the smoothed maximum unambiguously wherein the number of these several pulses can be predetermined or can depend on the heart rate of the subject. For instance, this number can increase with increasing heart rate. In an embodiment this number is 3 for a heart rate of 60/min and 10 for a heart rate of 200/min.

Preferentially, the blood pressure measurement is intended to be used in a sequence of measurements in rapid succession to allow for effective semi-continuous blood pressure monitoring such that the stress for the monitored individual, i.e. for the monitored subject, is minimized.

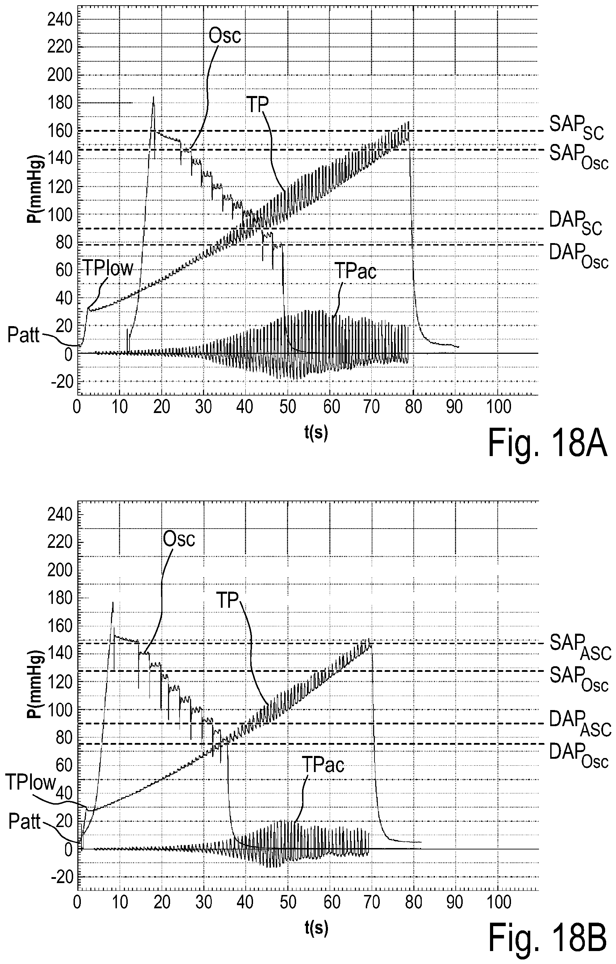

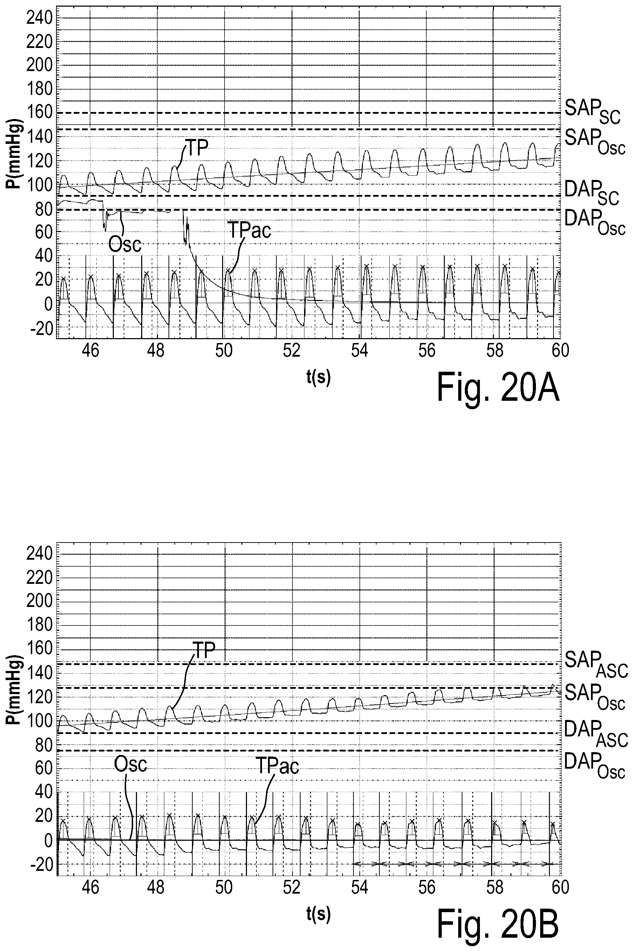

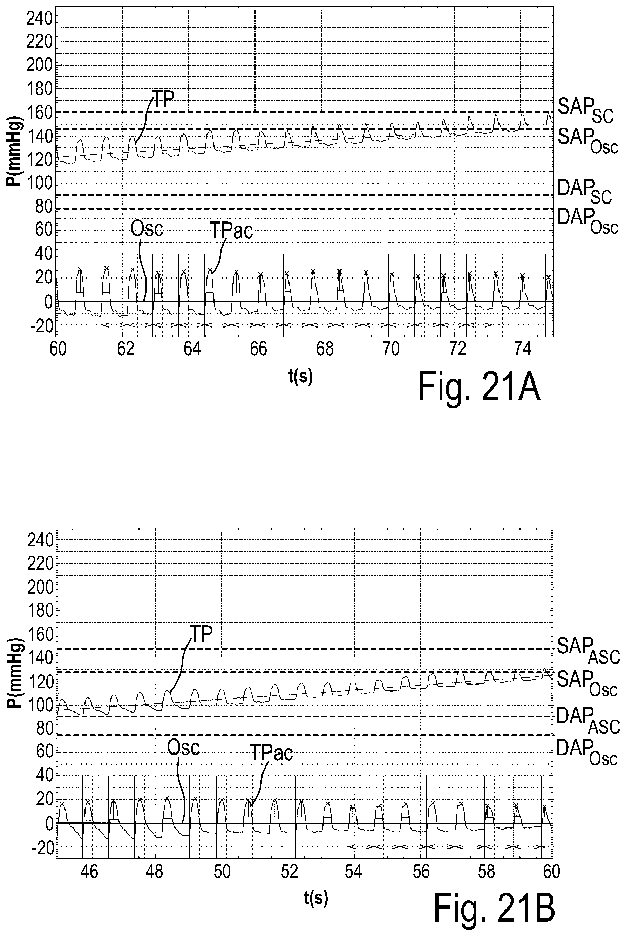

In the following the measurement quality achievable with a pressure measuring device 6 including an air-filled pressure sensing pad as described herein will be exemplarily indicated with reference to actually acquired measurement signals, and in comparison to corresponding measurement signals acquired with a pressure measuring device substantially identical to the pressure measuring device 6 described herein, which however includes a liquid-filled pressure sensing pad instead. As a further reference, measurement signals acquired with a conventional oscillometric blood pressure cuff are considered. While Figs. 18A, 19A, 20A, 21A show the measurement signals achieved with a liquid-filled pressure sensing pad together with the signals from the conventional oscillometric measurement, Figs. 18B, 19B, 20B, 21B correspond to Figs. 18A, 19A, 20A, 21A for the case of an air-filled pressure sensing pad. In particular, the measurement signals shown in Figs. 18B, 19B, 20B, 21B have been arrived at with a pressure measuring device in which the whole pressure sensing device, including the pressure transducer housing and the tube connecting the same with the pressure sensing pad, was air-filled.

Figs. 18A and 18B show the respective tissue pressure signals TP and their corresponding alternating components TPac known from their schematic illustration in Fig. 13 and the corresponding description above. Moreover, the respective signals Osc stem from conventional oscillometric blood pressure measurements that were carried out simultaneously to the acquisition of the respective signals TP. When comparing the tissue pressure signals TP in Figs. 18A and 18B with each other, the signal-dampening effect that has to be expected from the replacement of liquid by air in the signal transmission path becomes evident from the relative size of the oscillation amplitudes. This is also confirmed by the respective alternating components TPac. However, somewhat surprisingly, the dampening effect is rather little. And yet more importantly, the waveform of the pressure pulses is identical to the case of a liquid-filled pressure sensing pad.

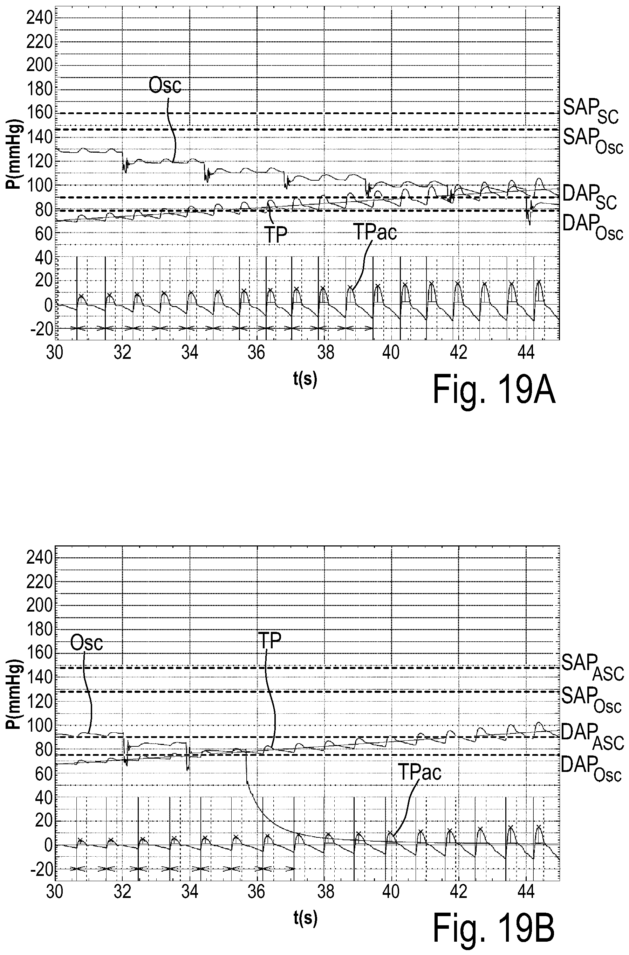

Figs. 19A, 20A, 21A show sections of the signals from Fig. 18A around DAP, MAP and SAP, respectively, and Figs. 19B, 20B, 21B show sections of the signals from Fig. 18B around DAP, MAP and SAP, respectively, wherein MAP stands for the mean arterial blood pressure, which may be calculated from DAP and SAP as MAP = DAP + (SAP - DAP) / 3. Hence, Figs. 19A to 21B zoom in on particularly relevant parts of the signals already shown in Figs. 18A and 18B.

For quantitatively comparing signal qualities, a first parameter that may be considered is TPPmx, which refers to the maximum TPP, i.e. the maximum amplitude across all respectively measured tissue pressure pulses. For this parameter, in a group of six subjects and with an adult shell size (for medial extremity circumference between 26 cm and 34cm) it was found that

$$\text{TPPmx (ASC)/TPPmx(SC)} = 15.4 \text{ mmHg}/20.2 \text{ mmHg} = 81.8\ \%, \qquad (4)$$

wherein ASC refers to the signal acquired with an air-filled pressure sensing pad (i.e., the "air shell cuff") and SC refers to the signal acquired with a liquid-filled pressure sensing pad (i.e., a known, liquid-based "shell cuff"). This nomenclature will be maintained in the following. Hence, while, as already mentioned above, ASC yielded identical pulse waveforms as the SC, it exhibited a TPPmx amplitude of approximately 82% as compared to the hydraulic sensing system SC, in this exemplary measurement.

However, since TPPmx is dependent on the level of blood pressure, the parameter TFmx should be considered the better parameter to compare intermittent non-invasive blood pressure (NIBP) measurement solutions like SC, ASC and conventional oscillometric approaches from each other. The conventional oscillometric pressure measurement will be abbreviated NIBPosc in the following.

Using the parameter TFmx, it can be measured which percentage of intra-arterial pulsations can be detected through the skin depending on the technology used. It can be defined as

$$\text{TFmx} = \text{TPPmx} / (\text{SAP - DAP}). \qquad (5)$$

Hence, the parameter TFmx normalizes the maximum non-invasive pulsation amplitude by the pulse pressure and hence provides a quality indicator that is independent of the pulse pressure and hence also the blood pressure to some degree.

For the exemplary measurement carried out, the following values were found:

$$\text{TFmx(SC)} = 35.1\ \%, \qquad \text{TFmx(ASC)} = 31.3\ \%, \ \text{TFmx(NIBP)} = 9.5\ \% \qquad (6a)$$

$$\text{TFmx(ASC)/TFmx(SC)} = 31.3/35.1 = 89\ \% \qquad (6b)$$

$$\text{TFmx(NIBPosc)/TFmx(SC)} = 9.5/35.1 = 27\ \% \qquad (6c)$$

$$\text{TFmx(NIBPosc)/TFmx(ASC)} = 9.5/31.3 = 30\ \% \qquad (6d)$$

The quotient TFmx(NIBPosc)/TFmx of any of the shell-based pressure measuring devices referred to herein on an adult upper arm is quite high in any normal BMI subject, however it is getting much lower (i.e., worse) in high BMI subjects. In other words, the advantages of the shell-based pressure measuring devices over conventional non-invasive oscillometric blood pressure measurement devices increase with increasing BMI.

From the above values it will be appreciated that, in the exemplary measurements carried out, the loss in signal quality caused by the change to an air-filled pressure sensing pad was relatively little as compared to the superiority of the signal quality of the shell-based solutions referred to herein over the conventional oscillometric one.

A further indicator for assessing the measurement quality is the efficacy, which measures how efficiently the pressure applied to the cuff, i.e. the actuator pressure Pact during inflation, translates to the pressure applied to the tissue, as measurable in terms of the clamping pressure TPcl. Hence, the efficacy, abbreviated as Eff, may be defined as

$$Eff = Pact' \, / \, TPcl', \qquad\qquad (7)$$

wherein Pact' indicates the slope of Pact and TPcl' indicates the slope of TPcl. In the exemplary measurement carried out,

$$Eff(ASC)/Eff(SC) = 82.2/94.3 = 114.8\% \qquad\qquad (8)$$

was found, meaning that filling the pressure sensing pad with air led to an increase in the efficacy by 14.8% as compared to the case of a liquid filled pressure sensing pad.

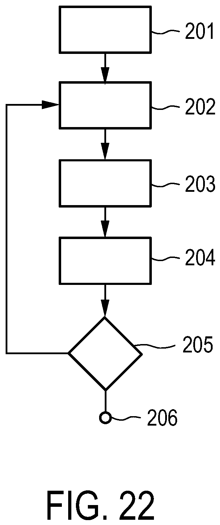

In the following an embodiment of a method for determining a physiological parameter of a subject will be exemplary described with reference to a flowchart shown in Fig. 22.

In step 201, the pressure measuring device 6 is arranged around the body part 5 of the subject. In step 202, a pressure signal of the subject, i.e. the tissue pressure TP, is measured by using the pressure measuring device 6, wherein the measured pressure signal is indicative of blood pulsations. In particular, the pressure application element 60, especially the fluid bag 88, applies increasing or decreasing pressure to the body part 5, while the tissue pressure TP is measured at the pressure sensing area 72 of the shell 4. In step 203, the physiological parameter, for instance the blood pressure, is determined based on the measured pressure signal by the processor 3 of the apparatus 1 for determining a physiological parameter of the subject. In step 204, the determined physiological parameter can be output to the medical personnel via an output unit like the display 22.

In step 205, it is determined whether an abort criterion is fulfilled. If this is the case, the method stops in step 206, otherwise it proceeds with step 202. Thus, the method can be carried out in a loop for continuously measuring the tissue pressure and for continuously monitoring the physiological parameter like the blood pressure over time in several measurement cycles, until the abort criterion is fulfilled. For instance, the measurement and monitoring can be interrupted, if a user like a physician has input a corresponding command into the apparatus via an input unit like a keyboard, a computer mouse, a touchpad, et cetera.

Since steps 201, 202, 205 and 206 provide the measured pressure signal until the abort criterion is fulfilled, these steps could also be regarded as being steps of a pressure measuring method for measuring pressure of a subject.

The pressure measuring device allows for an at least partially pneumatic coupling of the pressure measurement, wherein a transcutaneous high-fidelity recording of the arterial pressure waveform can be possible, in which even the closure of the aortic valve, i.e. the dicrotic notch, can be visible.

Although in above described embodiments the determined physiological parameter is the blood pressure, also another physiological parameter can be determined like the respiration rate, the heart rate, the fluid responsiveness, the cardiac output and contraction force, the arterial resistance, et cetera. For more details regarding the measurement of the different physiological parameters based on the measured tissue pressure reference is made to, for instance, WO 2018/210931 A1, WO 2019/211210 A1, WO 2021/255064 A1 and WO 2022/117471 A1, which are herewith incorporated by reference.

Although in above described embodiments features are determined by using the TPac pulses as processed pressure pulses, the features can also be determined by using directly the measured pressure pulses, i.e., for instance, the TP pulses. It is also possible to process the pressure pulses in another way, i.e., for example, not by subtracting the mean TP value for determining the TPac pulses. For instance, for the respective measured pressure pulse the pressure values at t.start and t.stop can be connected by a straight line and this straight line can be subtracted from the respective measured pressure pulse, in order to determine a processed pressure pulse.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of features of pulses, of certain curves, of the physiological parameter, et cetera performed by one or several units or devices can be performed by any other number of units or devices. The calculations and determinations and/or the control of the apparatus for determining the physiological parameter of the subject in accordance with the method for determining the physiological parameter of the subject and/or the control of the pressure measuring device in accordance with the pressure measuring method can be implemented as program code means of a computer and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a pressure measuring device configured to surround a subject's body part, wherein the pressure measuring device comprises a) a pressure application element configured to apply pressure to the body part, and b) a shell arranged to be located between the pressure application element and the body part, when the pressure measuring device surrounds the body part. Furthermore, the pressure measuring device comprises c) a pressure sensing device comprising a fluid-filled pressure sensing pad arranged in a pressure sensing area extending, as measured in a proximal-distal direction, along a middle of an inner surface of the shell, wherein the pressure sensing device is configured to measure a pressure signal indicative of blood pulsations in the subject. The pressure measuring device allows for an accurate measurement of a physiological parameter like blood pressure, even if the subject is severely obese.

**Claims**

1. A pressure measuring device (6) configured to surround a subject's body part (5), wherein the pressure measuring device comprises:

   - a pressure application element (88) configured to apply pressure to the body part (5),
   - a shell (4) arranged to be located between the pressure application element (88) and the body part (5), to extend along the body part (5) in a proximal-distal direction (83) and to extend around the body part (5) in a circumferential direction, when the pressure measuring device (6) surrounds the body part (5), and
   - a pressure sensing device configured to measure a pressure signal indicative of blood pulsations in the subject, the pressure sensing device comprising a fluid-filled pressure sensing pad (61) arranged in a pressure sensing area (72) extending along a middle of an inner surface of the shell (4), the middle being defined with respect to the proximal-distal direction (83).

2. The pressure measuring device as defined by claim 1, wherein the body part (5) is a distal body part, preferably a proximal part of a forearm or a distal part of a lower leg.

3. The pressure measuring device as defined by any of the preceding claims, wherein a) a distance between a proximal edge of the pressure sensing area (72) and a proximal edge of the shell (4) and b) a distance between a distal edge of the pressure sensing area (72) and a distal edge of the shell (4) are both in a range between 20% and 40% of an entire dimension of the shell (4) in the proximal-distal direction.

4. The pressure measuring device as defined by any of the preceding claims, wherein the shell (4) has a dimension between 80 mm and 100 mm in the proximal-distal direction (83) and a dimension of at least 360 mm in the circumferential direction.

5. The pressure measuring device as defined by any of the preceding claims, wherein the pressure sensing area (72) has a dimension between 70 mm and 215 mm in the circumferential direction and a dimension between 30 mm and 40 mm in the proximal-distal direction (83).

6. The pressure measuring device as defined by any of the preceding claims, wherein the pressure sensing area (72) extends into a circumferential end portion of the shell (4), preferably up to a distance of at least 5 mm between the pressure sensing area (72) and an edge of the circumferential end portion of the shell (4).

7. The pressure measuring device as defined by any of the preceding claims, wherein, when no external force acts upon the shell (4), the shell (4) has the form of a cylinder that is tapered in proximal-distal direction (83) at an angle between 4° and 5° relative to a non-tapered cylinder, preferably an angle of 4.5°.

8. The pressure measuring device as defined by any of the preceding claims, wherein the shell (4) is segmented in circumferential direction, wherein at least one segment (44) of the shell (4) is thinner and/or more flexible than at least one other segment of the shell (4).

9. The pressure measuring device as defined by any of the preceding claims, wherein the fluid with which the pressure sensing pad (61) is filled is air.

10. The pressure measuring device as defined by any of the preceding claims, wherein the pressure sensing device comprises a tube (62) for guiding pressure from the pressure sensing pad (61) to a pressure transducer, wherein the tube (62) is filled with air.

11. The pressure measuring device as defined by any of the preceding claims, wherein the pressure sensing device comprises a pressure transducer (69) located within the pressure sensing pad (61) or between the pressure sensing pad (61) and the inner surface of the shell (4).

12. An apparatus (1) for determining a physiological parameter of a subject, wherein the apparatus comprises:

    - a pressure measuring device (6) as defined by any of the preceding claims for measuring a pressure signal of the subject over a time, wherein the measured pressure signal is indicative of blood pulsations,
    - a processor (3) configured to determine the physiological parameter based on the measured pressure signal.

13. A pressure measuring method for measuring pressure of a subject, wherein the method comprises:

    - arranging a pressure measuring device (6) as defined in any of claims 1 to 11 around a body part (5) of the subject,
    - applying pressure to the body part (5) by the pressure application element (88) of the pressure measuring device (6), and
    - measuring a pressure signal of the subject, wherein the measured pressure signal is indicative of blood pulsations, by the pressure sensing device of the pressure measuring device (6).

14. A method for determining a physiological parameter of a subject, wherein the method comprises:

    - measuring a pressure signal of the subject over a time, wherein the measured pressure signal is indicative of blood pulsations, as defined by claim 13,
    - determining the physiological parameter based on the measured pressure signal by a processor (3).

15. A computer program for determining a physiological parameter of a subject, wherein the computer program comprises program code means for causing the apparatus (1) for determining a physiological parameter as defined by claim 13 to carry out the steps of the method as defined by claim 14.

Fig. 1

Fig. 2

Fig. 3A

Fig. 3B

Fig. 4A

Fig. 4B

Fig.5

Fig. 6

Fig. 7

Fig. 8

Fig. 9

68

Fig. 10

Fig. 11

Fig. 12

Fig. 13

Fig. 14

Fig. 15

Fig. 16

Fig. 17

Fig. 18A

Fig. 18B

Fig. 19A

Fig. 19B

Fig. 20A

Fig. 20B

Fig. 21A

Fig. 21B

FIG. 22

Europäisches
Patentamt
European
Patent Office
Office européen
des brevets

# EUROPEAN SEARCH REPORT

**Application Number**

EP 24 18 1804

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/359446 A1 (PFEIFFER ULRICH [DE] ET AL) 17 December 2015 (2015-12-17) * abstract; figure 3 * * paragraph [0058] * * paragraph [0084] * * paragraph [0090] * * paragraph [0115] * | 1-15 | INV. A61B5/021 A61B5/022 A61B5/00 |
| X | US 2019/090762 A1 (SAWANOI YUKIYA [JP] ET AL) 28 March 2019 (2019-03-28) * abstract; figure 12 * * paragraph [0036] - paragraph [0037] * * paragraph [0089] - paragraph [0092] * | 1-15 | |
| X | US 2023/218182 A1 (PFEIFFER ULRICH JOACHIM [NL] ET AL) 13 July 2023 (2023-07-13) * abstract; figure 2 * | 1-15 | |
| A | US 2010/106029 A1 (FRADEN JACOB [US]) 29 April 2010 (2010-04-29) * abstract * * figures 18,22 * | 1-15 | |
| A | US 8 998 817 B2 (PFEIFFER ULRICH [DE]; THOMAMUELLER TOBIAS [DE] ET AL.) 7 April 2015 (2015-04-07) * abstract * * figure 4 * | 1-15 | **TECHNICAL FIELDS SEARCHED (IPC)** A61B |
| A | EP 4 162 863 A1 (KONINKLIJKE PHILIPS NV [NL]) 12 April 2023 (2023-04-12) * abstract * * paragraph [0039] * | 1-15 | |
| A | WO 2021/115173 A1 (HUAWEI TECH CO LTD [CN]) 17 June 2021 (2021-06-17) * abstract * * figure 3 * | 1-15 | |

The present search report has been drawn up for all claims

| Place of search | Date of completion of the search | Examiner |
|---|---|---|
| The Hague | 20 November 2024 | Dhondt, Erik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 1804

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2024

| Patent document cited in search report | | Publication date | Patent family member(s) | | Publication date |
|---|---|---|---|---|---|
| US 2015359446 | A1 | 17-12-2015 | CN | 105228516 A | 06-01-2016 |
| | | | CN | 110811586 A | 21-02-2020 |
| | | | JP | 6360838 B2 | 18-07-2018 |
| | | | JP | 2016509516 A | 31-03-2016 |
| | | | US | 2015359446 A1 | 17-12-2015 |
| | | | US | 2022257130 A1 | 18-08-2022 |
| | | | WO | 2014121805 A1 | 14-08-2014 |
| | | | WO | 2014121945 A1 | 14-08-2014 |
| US 2019090762 | A1 | 28-03-2019 | CN | 109195514 A | 11-01-2019 |
| | | | DE | 112017002688 T5 | 07-03-2019 |
| | | | JP | 6610433 B2 | 27-11-2019 |
| | | | JP | 2017209434 A | 30-11-2017 |
| | | | US | 2019090762 A1 | 28-03-2019 |
| | | | WO | 2017203958 A1 | 30-11-2017 |
| US 2023218182 | A1 | 13-07-2023 | CN | 115701937 A | 14-02-2023 |
| | | | EP | 3925527 A1 | 22-12-2021 |
| | | | EP | 4167841 A1 | 26-04-2023 |
| | | | JP | 2023529974 A | 12-07-2023 |
| | | | US | 2023218182 A1 | 13-07-2023 |
| | | | WO | 2021255064 A1 | 23-12-2021 |
| US 2010106029 | A1 | 29-04-2010 | CN | 101730502 A | 09-06-2010 |
| | | | EP | 2139387 A1 | 06-01-2010 |
| | | | JP | 2010522610 A | 08-07-2010 |
| | | | KR | 20100027093 A | 10-03-2010 |
| | | | TW | 200843697 A | 16-11-2008 |
| | | | US | 2010106029 A1 | 29-04-2010 |
| | | | WO | 2008121454 A1 | 09-10-2008 |
| US 8998817 | B2 | 07-04-2015 | NONE | | |
| EP 4162863 | A1 | 12-04-2023 | CN | 118102969 A | 28-05-2024 |
| | | | EP | 4162863 A1 | 12-04-2023 |
| | | | EP | 4415603 A1 | 21-08-2024 |
| | | | JP | 2024536364 A | 04-10-2024 |
| | | | WO | 2023061704 A1 | 20-04-2023 |
| WO 2021115173 | A1 | 17-06-2021 | CN | 112971751 A | 18-06-2021 |
| | | | EP | 4059415 A1 | 21-09-2022 |
| | | | JP | 7404539 B2 | 25-12-2023 |
| | | | JP | 2023505867 A | 13-02-2023 |
| | | | US | 2023019122 A1 | 19-01-2023 |
| | | | WO | 2021115173 A1 | 17-06-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018210931 A1
- WO 2019211210 A1
- WO 2021255064 A1
- WO 2022117471 A1

**Non-patent literature cited in the description**

- **J. BRIEGEL et al.** Clinical Evaluation of a High-fidelity Upper Arm Cuff to Measure Arterial Blood Pressure during Noncardiac Surgery. *Anesthesiology*, 2020, vol. 133, 997-1006